# EUROPEAN PATENT SPECIFICATION

(11) **EP 3 152 295 B1**
(45) Date of publication and mention of the grant of the patent: **29.07.2020**
(21) Application number: 15802756.5
(22) Date of filing: 05.06.2015
(51) Int. Cl.: C12N 5/00, C12N 15/00, A01N 63/00, C12N 5/074, A61K 35/17, C12N 15/86, A61K 35/12

(54) **A NOVEL AND EFFICIENT METHOD FOR REPROGRAMMING IMMORTALIZED LYMPHOBLASTOID CELL LINES TO INDUCED PLURIPOTENT STEM CELLS**
NEUARTIGES UND EFFIZIENTES VERFAHREN ZUR UMPROGRAMMIERUNG IMMORTALISIERTER LYMPHOBLASTOIDER ZELLLINIEN ZU INDUZIERTEN PLURIPOTENTEN STAMMZELLEN
PROCÉDÉ NOUVEAU ET EFFICACE POUR REPROGRAMMER DES LIGNÉES CELLULAIRES LYMPHOBLASTOÏDES IMMORTALISÉES EN CELLULES SOUCHES PLURIPOTENTES INDUITES

(30) Priority: 05.06.2014 US 201462008198 P
(43) Date of publication of application: 12.04.2017
(73) Proprietor: Cedars-Sinai Medical Center, Los Angeles, CA 90048 (US)
(72) Inventor: SAREEN, Dhruv, Porter Ranch, California 91326 (US); ORNELAS, Loren A., Los Angeles, California 90032 (US); BARRETT, Robert, Los Angeles, California 90046 (US)
(74) Representative: HGF
(86) International application number: PCT/US2015/034532
(87) International publication number: WO 2015/188131

(56) References cited:
- WO-A1-2013/062140
- WO-A2-2011/159684
- US-A1- 2012 009 676
- US-A1- 2012 196 360
- US-B2- 8 257 941
- S. M. CHOI ET AL: "Reprogramming of EBV-immortalized B-lymphocyte cell lines into induced pluripotent stem cells", BLOOD, vol. 118, no. 7, 31 May 2011 (2011-05-31), pages 1801-1805, XP055083381, ISSN: 0006-4971, DOI: 10.1182/blood-2011-03-340620
- ZHAOHUI YE ET AL: "Hematopoietic cells as sources for patient-specific iPSCs and disease modeling", CELL CYCLE, vol. 10, no. 17, 1 September 2011 (2011-09-01), pages 2840-2844, XP055092521, ISSN: 1538-4101, DOI: 10.4161/cc.10.17.17180
- D. RAJESH ET AL: "Human lymphoblastoid B-cell lines reprogrammed to EBV-free induced pluripotent stem cells", BLOOD, vol. 118, no. 7, 27 June 2011 (2011-06-27) , pages 1797-1800, XP055083379, ISSN: 0006-4971, DOI: 10.1182/blood-2011-01-332064
- ROBERT BARRETT ET AL: "Reliable Generation of Induced Pluripotent Stem Cells From Human Lymphoblastoid Cell Lines : Reliable iPSC Generation From LCLs", STEM CELLS TRANSLATIONAL MEDICINE : SCTM, vol. 3, no. 12, 8 October 2014 (2014-10-08), pages 1429-1434, XP055403729, Durham ISSN: 2157-6564, DOI: 10.5966/sctm.2014-0121

### SEQUENCE LISTING

<110> CEDARS-SINAI MEDICAL CENTER
   SAREEN, Dhruv
   ORNELAS, Loren A.
   BARRETT, Robert
<120> A NOVEL AND EFFICIENT METHOD FOR REPROGRAMMING IMMORTALIZED LYMPHOBLASTOID CELL LINES TO INDUCED PLURIPOTENT STEM CELLS
<130> 065472-000436WO00
<150> 62/008,198
   <151> 2014-06-05
<160> 48
<170> PatentIn version 3.5
<210> 1
   <211> 23
   <212> DNA
   <213> Homo sapiens
<400> 1
   ccccagggcc ccattttggt acc 23
<210> 2
   <211> 26
   <212> DNA
   <213> Homo sapiens
<400> 2
   acctcagttt gaatgcatgg gagagc 26
<210> 3
   <211> 20
   <212> DNA
   <213> Homo sapiens
<400> 3
   cattcaaact gaggtaaggg 20
<210> 4
   <211> 22
   <212> DNA
   <213> Homo sapiens
<400> 4
   tagcgtaaaa ggagcaacat ag 22
<210> 5
   <211> 24
   <212> DNA
   <213> Homo sapiens
<400> 5
   ttcacatgtc ccagcactac caga 24
<210> 6
   <211> 26
   <212> DNA
   <213> Homo sapiens
<400> 6
   tcacatgtgt gagaggggca gtgtgc 26
<210> 7
   <211> 24
   <212> DNA
   <213> Homo sapiens
<400> 7
   ttcacatgtc ccagcactac caga 24
<210> 8
   <211> 22
   <212> DNA
   <213> Homo sapiens
<400> 8
   tttgtttgac aggagcgaca at 22
<210> 9
   <211> 24
   <212> DNA
   <213> Homo sapiens
<400> 9
   acccatcctt cctgcccgat caga 24
<210> 10
   <211> 24
   <212> DNA
   <213> Homo sapiens
<400> 10
   ttggtaatgg agcggcggga cttg 24
<210> 11
   <211> 23
   <212> DNA
   <213> Homo sapiens
<400> 11
   ccacctcgcc ttacacatga aga 23
<210> 12
   <211> 22
   <212> DNA
   <213> Homo sapiens
<400> 12
   tagcgtaaaa ggagcaacat ag 22
<210> 13
   <211> 26
   <212> DNA
   <213> Homo sapiens
<400> 13
   gcgaacccaa gacccaggcc tgctcc 26
<210> 14
   <211> 25
   <212> DNA
   <213> Homo sapiens
<400> 14
   cagggggtct gctcgcaccg tgatg 25
<210> 15
   <211> 21
   <212> DNA
   <213> Homo sapiens
<400> 15
   ggctgagaag aggatggcta c 21
<210> 16
   <211> 22
   <212> DNA
   <213> Homo sapiens
<400> 16
   tttgtttgac aggagcgaca at 22
<210> 17
   <211> 25
   <212> DNA
   <213> Homo sapiens
<400> 17
   agccatatgg tagcctcatg tccgc 25
<210> 18
   <211> 30
   <212> DNA
   <213> Homo sapiens
<400> 18
   tcaattctgt gcctccggga gcagggtagg 30
<210> 19
   <211> 25
   <212> DNA
   <213> Homo sapiens
<400> 19
   agccatatgg tagcctcatg tccgc 25
<210> 20
   <211> 22
   <212> DNA
   <213> Homo sapiens
<400> 20
   tagcgtaaaa ggagcaacat ag 22
<210> 21
   <211> 21
   <212> DNA
   <213> Homo sapiens
<400> 21
   cctggaggag aagaggaaag a 21
<210> 22
   <211> 21
   <212> DNA
   <213> Homo sapiens
<400> 22
   ttgaggacct ctgtgtattt g 21
<210> 23
   <211> 21
   <212> DNA
   <213> Homo sapiens
<400> 23
   tgctgtctcc atgtttgatg t 21
<210> 24
   <211> 21
   <212> DNA
   <213> Homo sapiens
<400> 24
   tctctgctcc ccacctctaa g 21
<210> 25
   <211> 21
   <212> DNA
   <213> Homo sapiens
<400> 25
   atcagggcca agacatagag a 21
<210> 26
   <211> 20
   <212> DNA
   <213> Homo sapiens
<400> 26
   gccaatgcaa cttggacgtt 20
<210> 27
   <211> 24
   <212> DNA
   <213> Homo sapiens
<400> 27
   catagaagaa gaagaggatg aaga 24
<210> 28
   <211> 24
   <212> DNA
   <213> Homo sapiens
<400> 28
   gtagggattc gagggaatta ctga 24
<210> 29
   <211> 19
   <212> DNA
   <213> Homo sapiens
<400> 29
   atggaacacg accttgaga 19
<210> 30
   <211> 20
   <212> DNA
   <213> Homo sapiens
<400> 30
   tgagcaggat gaggtctagg 20
<210> 31
   <211> 20
   <212> DNA
   <213> Homo sapiens
<400> 31
   cacctcaacc tggagacaat 20
<210> 32
   <211> 20
   <212> DNA
   <213> Homo sapiens
<400> 32
   tgaagcaggc gtggtttcaa 20
<210> 33
   <211> 20
   <212> DNA
   <213> Homo sapiens
<400> 33
   tcgggggtgt tagagacaac 20
<210> 34
   <211> 20
   <212> DNA
   <213> Homo sapiens
<400> 34
   ttccacgagg gtagtgaacc 20
<210> 35
   <211> 20
   <212> DNA
   <213> Homo sapiens
<400> 35
   accacagtcc atgccatcac 20
<210> 36
   <211> 20
   <212> DNA
   <213> Homo sapiens
<400> 36
   tccaccaccc tgttgctgta 20
<210> 37
   <211> 20
   <212> DNA
   <213> Homo sapiens
<400> 37
   tccttctacg gacggaactg 20
<210> 38
   <211> 20
   <212> DNA
   <213> Homo sapiens
<400> 38
   agaaatgcct gaggaaagca 20
<210> 39
   <211> 20
   <212> DNA
   <213> Homo sapiens
<400> 39
   gattcctcct ccaccctcac 20
<210> 40
   <211> 23
   <212> DNA
   <213> Homo sapiens
<400> 40
   caatattctg cctggcctgg atg 23
<210> 41
   <211> 21
   <212> DNA
   <213> Homo sapiens
<400> 41
   ccacacccac tcagagccat t 21
<210> 42
   <211> 20
   <212> DNA
   <213> Homo sapiens
<400> 42
   caccccacca ccaaaacctt 20
<210> 43
   <211> 24
   <212> DNA
   <213> Homo sapiens
<400> 43
   cgagaggacc ccgtggatgc agag 24
<210> 44
   <211> 24
   <212> DNA
   <213> Homo sapiens
<400> 44
   ggcggccatc ttcagcttct ccag 24
<210> 45
   <211> 28
   <212> DNA
   <213> Homo sapiens
<400> 45
   gaatgctgca aactgaccac gctggaac 28
<210> 46
   <211> 28
   <212> DNA
   <213> Homo sapiens
<400> 46
   tggcattcaa gagggttttc agtctgga 28
<210> 47
   <211> 18
   <212> DNA
   <213> Homo sapiens
<400> 47
   ctatcatgct ggctgcct 18
<210> 48
   <211> 20
   <212> DNA
   <213> Homo sapiens
<400> 48
   ctacaacacc cttctcacag 20

## Description

### FIELD OF INVENTION

Described herein are methods and compositions related to regenerative medicine including the derivation of induced pluripotent stem cells (iPSCs) from lymphoblastoid cells, such methods and compositions provide a renewable source of transplant material.

### BACKGROUND

Pluripotent stem cells ("pSCs") present broad opportunities to generate therapeutic materials for use in regenerative medicine, as well as providing invaluable *in vitro* models for studying disease initiation and progression. One category of pSCs, induced pluripotent stem cells ("iPSCs"), possess the hallmark stem cell properties of self-renewal (i.e., immortal) and differentiation capacity into cells derived from all three embryonic germ layers (i.e., pluripotency). These cells can be obtained through "reprogramming", which involves dedifferentiation of cells from non-embryonic sources, such as adult somatic cells. The reprogramming process obviates potential ethical concerns over embryonic source material for other types such pSCs, such as embryonic stem cells ("ESCs"), while providing a further benefit of enabling potential patient-specific immunological incompatibility.

Breakthroughs in the reprogramming of somatic cells into iPSCs from primate sources were first reported by independent led groups led by Thomson (Yu et al. Science 318:1917-1920 (2007) and Yamanaka (Takahashi et al., Cell 131:861-872 (2007)). Both groups delivered and expressed cDNA into human somatic cells through the use of viral vectors expressing factors related to pluripotency ("reprogramming factors"). Interestingly, initial reports differed in the combinations of transgenes successfully used for reprogramming. The Yamanaka group relied upon Oct-4, Sox-2, c-Myc and Klf-4 (i.e., "Yamanaka factors"), while the Thomson group utilized Oct-4, Sox-2, Nanog, and Lin-28 (i.e., "Thomson factors"). Despite the difference in choice of reprogramming factors, their delivery into, and expression by, somatic cells allowed acquisition of pSC-specific characteristics, including characteristic stem cell colony morphology, proliferation capacity and pluripotency, as well as proper gene and surface marker expression.

These initial reports were followed by attempts to obviate concerns over the use of integrative viral delivery systems by the Yamanaka and Thomson groups. Some advances were provided by successful ectopic expression of reprogramming factors, apparently without disturbing capacity to differentiate into cells of various lineages. Others eliminated use of potential proto-oncogenic factors, such as c-Myc, which were subsequently identified as not strictly necessary for reprogramming. Naturally, there is a high degree in heterogeneity in existing reprogramming studies, given the apparently wide opportunities to modify choice of delivery vectors, organization of reprogramming factors within delivery vectors, and variable choice in reprogramming factor combinations derived based on initial Yamanaka factors and/or Thomson factors. Despite the many efforts to improve reprogramming techniques, they have nevertheless been plagued by poor efficiency (often far less than 0.1%), irreproducibility, and limited extensibility across different target host cell types.

In addition to establishing robust reprogramming techniques, full realization of therapeutic goals for stem-cell regenerative medicine further requires consideration of the types of host cells that can serve as a resource for renewable regenerative material. Ideally, cells would possess not only the requisite plasticity for successful reprogramming and stability in subsequent propagation, but also provide advantages in clinical aspects, such as ease of isolation, storage, stability and maintenance. In this regard, lymphoblastoid B-cells and cell lines ("LCs") present an attractive choice for such uses, given the track record of using this particular cell type for long-term storage of a subject's genetic information, as well as demonstrated properties of multilineage plasticity and expansion capacity. The B-cell source material for LC cell lines are also readily obtainable from living subjects using a blood draw and can be easily genetically manipulated, such as transformation into cell lines following *in vitro* viral exposure.

Given the eventual therapeutic goal of generating patient-specific, immunocompatible biological material, there is a great need in the art to establish a robust and reproducible means for reprogramming cells, along with identifying sources of therapeutic materials suitable for eventual clinical application. Such improved methods would need to possess high efficiency of reprogramming, consistent reproduction, and be readily extendible to a variety of cell types.

Described herein are improved techniques for establishing highly efficient, reproducible reprogramming using non-integrating episomal plasmid vectors, including generation of iPSCs from lymphoblastoid B-cells and lymphoblastoid B-cell lines.

### BRIEF DESCRIPTION OF FIGURES

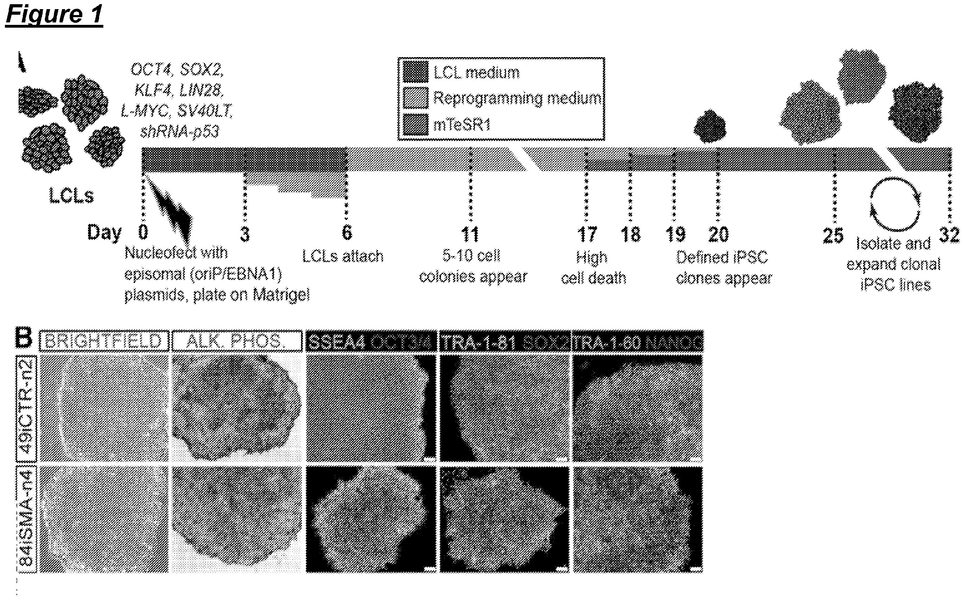

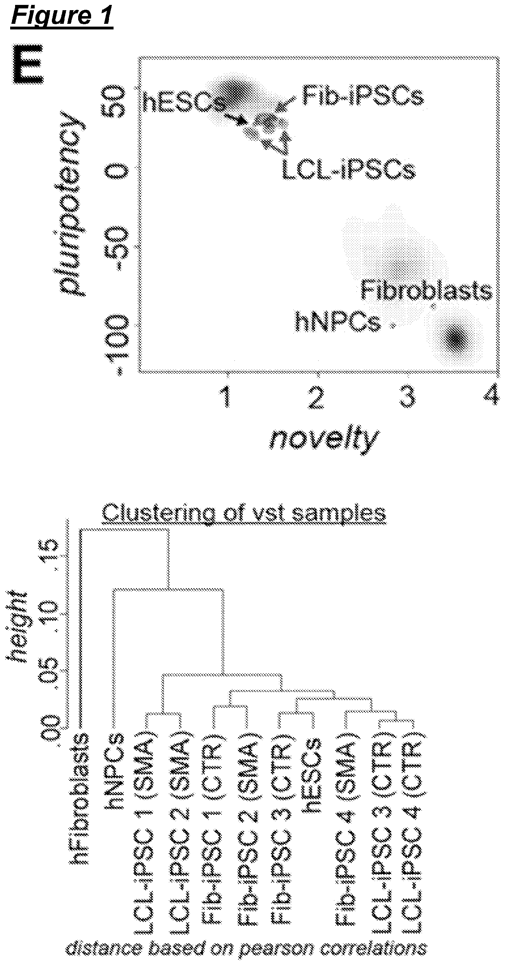

**Figure 1(A) to Figure 1 (I)****.** Generation and characterization of LCL-iPSCs. **(A):** Schematic depicting the episomal reprogramming process and timeline of iPSC generation from LCLs. **(B):** Bright-field images of the reprogrammed iPSC colonies from control and SMA LCLs showa high nuclear-to-cytoplasmic ratio and are alkaline phosphatase-positive and immunopositive for the surface antigens, SSEA4, TRA-1-60 and TRA-1-81 and nuclear pluripotency markers OCT3/4, SOX2, and NANOG. Scale bar = 75mm. **(C):** Flowcytometry analysis of representative clones from the 49iCTR (49iCTR-n2) and84iSMA (84iSMAn4) lines showing 96%of cells were immunopositiveforSSEA4andOCT3/4. **(D):** All the LCL-iPSC lines maintained anormal G-band karyotype as shown from representative lines. **(E):** Gene chip- and bioinformatics-based PluriTest characterization of eight LCL- or Fib-iPSC lines. **(F):** Quantitative reverse transcription-polymerase chain reaction (PCR) analyses of POU5F1 (OCT4), SOX2, LIN28, L-MYC, and KLF4 expression in 49iCTR and 84iSMA LCL-iPSCs relative to H9 hESCs. **(G):** Three sets of PCR primers were used to detect immunoglobulin heavy locus rearrangements occurring in the LCLs and iPSC lines. A clonal positive control and negative H9 hESCs control were included. **(H):** Epstein Barr virus-related genes (EBNA-1, EBNA-2, BZLF-1, LMP-1, and OriP) were analyzed by PCR analysis of genomic DNA obtained fromH9 ESCs, parental LCLs, and daughter iPSC lines. GAPDH was used a loading control. **(I):** PCR-restriction fragment-length polymorphism assay using Dde1 restriction enzyme digest illustrating maintenance of SMA genotype in SMA LCL-iPSCs as shown by undigested SMN, along with SMN1 and SMN2 in all four LCL-iPSC lines. GAPDH was used as a loading control. Abbreviations: ALK.PHOS., alkaline phosphatase; CDS, coding DNA sequence; CTR, control; Fib, fibroblast; hESC stem cells, human embryonic stem cells; hNPC, human neural progenitor cells; iPSC, induced pluripotent stem cell; LCL, lymphoblastoid cell line; Pla, plasmid; SMA, spinal muscular atrophy.

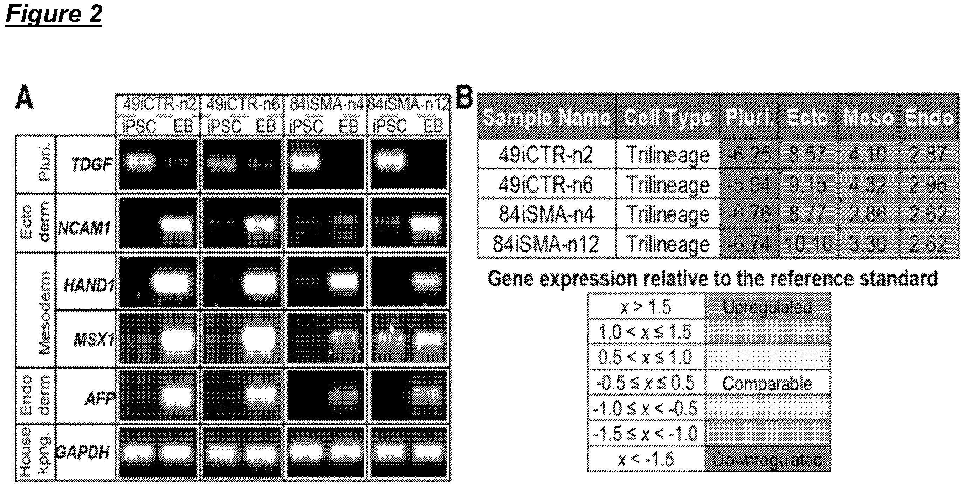

**Figure 2(A) to Figure 2(D)****.** Spontaneous and directed differentiation from LCL-iPSCs. **(A):** Spontaneous in vitro EB differentiation of all four LCL-iPSC lines illustrating iPSC (TDGF) and germ-layer (NCAM1, HAND1, MSX1, and AFP) specific gene expression. GAPDH was used as a loading control. **(B):** TaqMan human pluripotent stem cell scorecard table showing the trilineage potential of all four LCL-iPSCs. (C): Pairwise correlation coefficients, scatterplots, and expression pattern plots from selected genes in four gene groups (pluripotency, ectoderm, endoderm, and mesoderm) comparing EBs derived from all four LCL-iPSC lines. **(D):** Representative iPSCs directed to generate ectodermal (Sox2+/b3-tubulin+ neuronal cells), endodermal (CDX2+/FABP2+ intestinal enterocytes), and mesodermal (CD73+/Collagen type 1+ chondrocytes) cell types from the 49iCTRn2 line. Scale bar = 75 mm. Abbreviations: CTR, control; EB, embryoid body; Ecto, ectoderm; Endo, endoderm; iPSC, induced pluripotent stem cell; LCL, lymphoblastoid cell line; Meso, mesoderm; Pluri., pluripotency; SMA, spinal muscular atrophy.

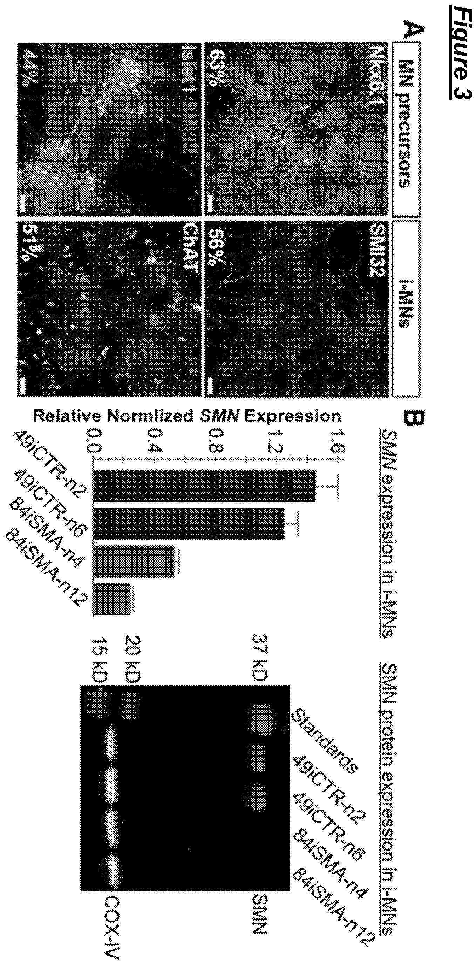

**Figure 3(A) to Figure 3(D)****.** LCL- and dermal fibroblast-derived iPSCs can similarly be directed to differentiate into disease-relevant cell types. **(A):** All LCL-iPSCs were capable of being directed to generate cells that are immunopositive for Nkx6.1, Islet1, SMI32, and ChAT, which represent different stages of MN development. The percentage of efficiency of iPSCs differentiated in to Nkx6.1 (63%66%), Islet1 (44%65%), SMI32 (56%63%), and ChAT (51% 6 4%) immunopositive cells was relative to total Hoechst-positive cells in the culture. Scale bar = 75mm. **(B):** Quantitative reverse transcription-polymerase chain reaction expression and Western blot analysis showing maintenance of depleted SMN in SMA patient LCL-iPSC directed to i-MNs, at both the mRNA and protein level, as compared with control i-MNs. COX-IV was used as a loading control. **(C):** iPSCs derived from either LCLs or dermal fibroblasts generate i-MNs at different developmental stages and general neurons (b3-tubulin) that are virtually indistinguishable fromeach other. Scale bar = 10 mm. The percentage of efficiency of iPSCs differentiated into cell types immunopositive for Islet1 (LCL:32%64%,fibroblast [fib]:33%63%),Nkx6.1 (LCL:14%62%; fib:12%63%), SMI32(LCL:56%63%; fib-iPSC:52%63%),ChAT(LCL:51%6 4%; fib: 47%65%), andb3-tubulin (LCL: 74%62%; fib: 72%65%) was relative to total Hoechst-positive cells in the culture. **(D):** LCL- or fib-iPSCs generate intestinal organoids that were all immunopositive and had similar morphologies for the intestinal transcription factor, CDX2, and the intestinal subtypes including goblet cells (Muc2+), enteroendocrine cells (CGA+), enterocytes (FABP2+), and Paneth cells (Lysozyme+). Scale bar = 75 mm. Abbreviations: ChAT, choline acetyltransferase; COX-IV, Cytochrome c Oxidase Subunit IV; CTR, control; i-MNs, iPSC-derived motoneurons; iPSC, induced pluripotent stem cell; LCL, lymphoblastoid cell line; MN, motoneuron; SMA, spinal muscular atrophy.

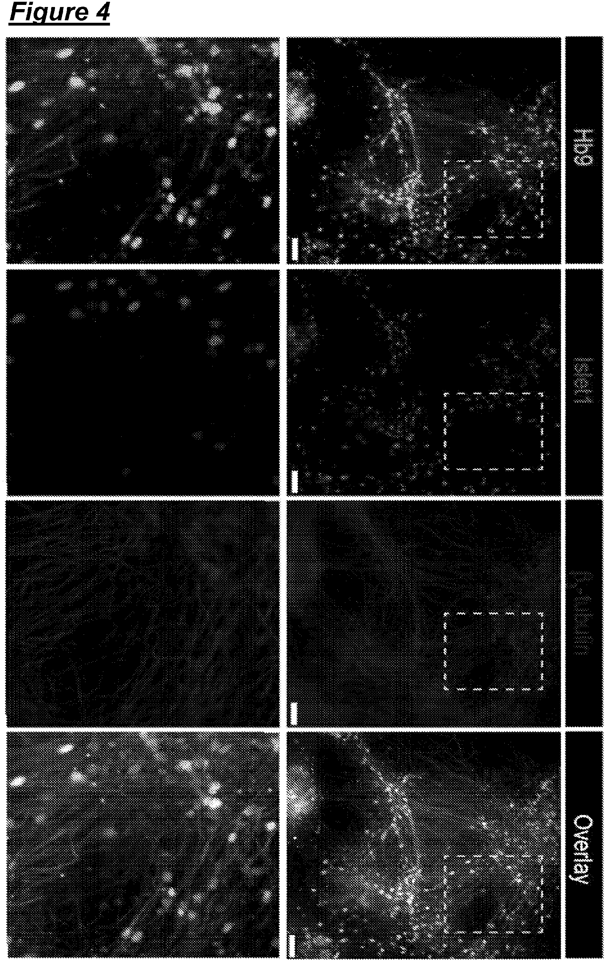

**Figure 4****.** LCL-derived iPSCs differentiate into Hb9 and Islet1-positive spinal motor neurons. LCL-iPSCs were directed to generate neuronal (β3-tubulin) cells that were also immunopositive for spinal motor neuron markers Hb9 and Islet1. The regions highlighted by the dashed lines in the upper panels illustrate Hb9, Islet1 and β3-tubulin immunopositive cells at higher magnification in the respective panels below. Scale bar, 75µm.

### SUMMARY OF THE INVENTION

Described herein in accordance with the invention is a method of generating lymphoid-cell line derived induced pluripotent stem cells, comprising providing a quantity of lymphoid cell line cells (LCLs), delivering a quantity of reprogramming factors into the LCLs wherein the reprogramming factors comprise short hairpin RNAs targeting p53 ("shRNA-p53"), culturing the LCLs in a reprogramming media for at least 7 days wherein the reprogramming media comprises small chemical induction molecules PD0325901, CHIR99021, HA-100, and A-83-01, and further culturing the LCLs in an induction media for at least 10 days, wherein delivering the reprogramming factors, culturing and further culturing generates lymphoid-cell line derived induced pluripotent stem cells. In other embodiments, delivering a quantity of reprogramming factors comprises nucleofection. In other embodiments, the reprogramming factors further comprise one or more factors selected from the group consisting of: Oct-4, Sox-2, Klf-4, c-Myc, Lin-28, SV40 Large T Antigen ("SV40LT"). In other embodiments, the reprogramming factors are encoded in one or more oriP/EBNA1 derived vectors. In other embodiments, the one or more oriP/EBNA1 derived vectors comprise pEP4 E02S ET2K, pCXLE-hOCT3/4-shp53-F, pCXLE-hSK, and pCXLE-hUL.. In other embodiments, culturing the LCLs in a reprogramming media is for at least 7, 8, 9, 10, 11, 12, 13, 14, 15, or 16 days. In other embodiments, culturing the LCLs in a reprogramming media is for 8 to 14 days. In other embodiments, further culturing the LCLs in an induction media is for at least 10, 11, 12, 13, 14, 15, 16, 17, 18, 19, 20, 21, 22, 23, 24, 25, 26, 27, 28, 29, or 30 days. In other embodiments, the induction media is a serum-free media. In other embodiments, the LCLs are have been isolated from a subject possessing a disease mutation. In other embodiments, the disease mutation is associated with a neurodegenerative disease, disorder and/or condition. In other embodiments, the disease mutation is associated with an inflammatory bowel disease, disorder, and/or condition.

Further described herein is an efficient method for generating induced pluripotent stem cells, comprising providing a quantity of lymphoid cells (LCs), delivering a quantity of reprogramming factors into the LCs, culturing the LCs in a reprogramming media for at least 7 days, and further culturing the LCs in an induction media for at least 10 days, wherein delivering the reprogramming factors, culturing and further culturing generates lymphoid-cell derived induced pluripotent stem cells. As described herein , the reprogramming factors may be encoded in pEP4 E02S ET2K, pCXLE-hOCT3/4-shp53-F, pCXLE-hSK, and pCXLE-hUL. As described herein , the reprogramming media may comprise PD0325901, CHIR99021, HA-100, and A-83-01. As described herein , the LCs may be in a reprogramming media for 8-14 days and the method may comprise further culturing the LCs in an induction media for 1-12 days.

Also disclosed herein is a pharmaceutical composition comprising a quantity of lymphoid-cell derived induced pluripotent stem cells generated by an efficient method for generating induced pluripotent stem cells, comprising providing a quantity of lymphoid cells (LCs), delivering a quantity of reprogramming factors into the LCs, culturing the LCs in a reprogramming media for at least 7 days, and further culturing the LCs in an induction media for at least 10 days, wherein delivering the reprogramming factors, culturing and further culturing generates lymphoid-cell derived induced pluripotent stem cells, and a pharmaceutically acceptable carrier.

Also disclosed herein is lymphoid-cell derived induced pluripotent stem cell line.

### DETAILED DESCRIPTION

. Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see Greenfield, Antibodies A Laboratory Manual 2nd ed., Cold Spring Harbor Press (Cold Spring Harbor NY, 2013); Köhler and Milstein, Derivation of specific antibody-producing tissue culture and tumor lines by cell fusion, Eur. J. Immunol. 1976 Jul, 6(7):511-9; Queen and Selick, *Humanized immunoglobulins,* U. S. Patent No. 5,585,089 (1996 Dec); and Riechmann et al., Reshaping human antibodies for therapy, Nature 1988 Mar 24, 332(6162):323-7.

One skilled in the art will recognize many methods and materials similar or equivalent to those described herein, which could be used in the practice of the present invention. For purposes of the present invention, the following terms are defined below.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise.

Patient-specific induced pluripotent stem cells ("iPSCs") hold great promise for many applications, including disease modeling to elucidate mechanisms involved in disease pathogenesis, drug screening, and ultimately regenerative medicine therapies. A frequently used starting source of cells for reprogramming has been dermal fibroblasts isolated from skin biopsies. However, numerous repositories containing lymphoblastoid cell lines ("LCLs") generated from a wide array of patients also exist in abundance. To date, this rich bioresource has been severely underused for iPSC generation. The Inventors first attempted to create iPSCs from LCLs using two existing methods but were unsuccessful.

Here the Inventors report a new and more reliable method for LCL reprogramming using episomal plasmids expressing pluripotency factors and p53 shRNA in combination with small molecules. The LCL-derived iPSCs ("LCL-iPSCs") exhibited identical characteristics to fibroblast derived iPSCs ("fib-iPSCs"), wherein they retained their genotype, exhibited a normal pluripotency profile, and readily differentiated into all three germ-layer cell types. As expected, they also maintained rearrangement of the heavy chain immunoglobulin locus. Importantly, the Inventors also show efficient iPSC generation from LCLs of patients with spinal muscular atrophy and inflammatory bowel disease. These LCL-iPSCs retained the disease mutation and could differentiate into neurons, spinalmotor neurons, and intestinal organoids, all of which were virtually indistinguishable from differentiated cells derived from fib-iPSCs. This method for reliably deriving iPSCs from patient LCLs paves the way for using invaluable worldwide LCL repositories to generate new human iPSC lines, thus providing an enormous bioresource for disease modeling, drug discovery, and regenerative medicine applications.

The ability to generate human iPSCs from adult somatic tissues has provided unprecedented opportunities for regenerative medicine. Furthermore, the differentiation of patient iPSCs into pathophysiologically affected cell types has provided tremendous insight into the mechanisms of various diseases. Dermal fibroblasts were the original and most widely reported cell type used for human iPSC derivation. A readily available, yet underutilized, bioresource for iPSC generation are lymphoblastoid cell lines. These lines are established by infecting resting peripheral B lymphocytes isolated from simple blood draws with Epstein Barr virus ("EBV") to give rise to actively proliferating LCLs.

Numerous LCLs are already available in well characterized worldwide repositories linked to patient clinical history, long-term genotype phenotype data, and molecular/functional studies. Despite the abundance and applicability of LCLs, there have been only two reports using LCLs to derive iPSCs. Unsuccessful attempts to derive iPSCs from LCLs following these protocols highlighted a need to optimize the system.

Here the Inventors report a successful and highly reproducible method of reprogramming human LCLs into iPSCs, from healthy individuals, as well as patients with spinal muscular atrophy ("SMA") and inflammatory bowel disease ("IBD"). The ability to reprogram patient LCLs for multitude of disease indications and differentiate them into disease-relevant cell types offers a remarkable opportunity to develop predictive cellular assays for disease modeling and drug discovery. Given that motoneurons ("MNs") and intestinal cells are particularly vulnerable in pathophysiology of SMA and IBD, respectively, the Inventors also demonstrate that LCL-iPSCs could effectively differentiate into these cell types.

As described, the Inventors have established improved techniques for highly efficient, reproducible reprogramming using non-integrating episomal plasmid vectors, including generation of iPSCs from lymphoblastoid B-cells and cell lines, the resulting reprogrammed pluripotent cells described herein as "LCL-iPSCs". Using the described techniques the inventors can achieved at least 10% conversion efficiency, representing at least 3-8 fold improvement compared to existing reprogramming studies.

Generally, different approaches for non-integrative reprogramming span at least categories: 1) integration-defective viral delivery, 2) episomal delivery, 3) direct RNA delivery, 4) direct protein delivery and 5) chemical induction. As described further herein, the adoption of episomal vectors allows for generation of iPSCs substantially free of the vectors used in their production, as episomal or similar vectors do not encode sufficient viral genome sufficient to give rise to infection or a replication-competent virus. At the same time, these vectors do possess a limited degree of self-replication capacity in the beginning somatic host cells. This self-replication capacity provides a degree of persistent expression understood to be beneficial in allowing the dedifferentiation process to initiate take hold in a target host cell.

One example of a plasmid vector satisfying these criteria includes the Epstein Barr oriP/Nuclear Antigen-1 ("EBNA1") combination, which is capable of limited self-replication and known to function in mammalian cells. As containing two elements from Epstein-Barr virus, oriP and EBNA1, binding of the EBNA1 protein to the virus replicon region oriP maintains a relatively long-term episomal presence of plasmids in mammalian cells. This particular feature of the oriP/EBNA1 vector makes it ideal for generation of integration-free iPSCs.

More specifically, persistent expression of reprogramming factor encoded in an oriP/EBNA1 vector occurs across multiple cell divisional cycles. Sufficiently high levels of reprogramming factors across several cell divisions allows for successful reprogramming even after only one infection. While sustained expression of reprogramming factors is understood to be beneficial during initial programming stages, otherwise unlimited constitutive expression would hamper subsequent stages of the reprogramming process. For example, unabated expression of reprogramming factors would interfere with subsequent growth, development, and fate specification of the host cells.

At the same time, a further benefit is the eventual removal of the reprogramming factor transgenes, as a small portion of episomes is lost per cell cycle. This is due to the asymmetric replication capacity of the host cell genome and episomal self-replication and it is estimated that approximately 0.5% of vector is lost per generation. Gradual depletion of plasmids during each cell division is inevitable following propagation leading to a population of integration-free iPSCs. The persistent, yet eventual abrogation of reprogramming factor expression on oriP/EBNA1 is highly coincident with the needs for different stages of the reprogramming process and eliminates the need for further manipulation steps for excision of the reprogramming factors, as has been attempted through use of transposons and excisable polycistronic lentiviral vector elements. Although oriP/EBNA1 has been applied by others in reprogramming studies, the reported efficiencies are extremely low (as few as 3 to 6 colonies per million cells nucleofected), which may be due, in-part, to reliance on large plasmids encoding multiple reprogramming factors (e.g., more than 12 kb), negatively impacting transfection efficiency.

In addition to these choices in vector designs, the specific combinations of reprogramming factors implemented in the literature have varied. As mentioned, reprogramming factors that have been used include pluripotency-related genes Oct-4, Sox-2, Lin-28, Nanog, Sall4, Fbx-15 and Utf-1. These factors, traditionally are understood be normally expressed early during development and are involved in the maintenance of the pluripotent potential of a subset of cells that will constituting the inner cell mass of the pre-implantation embryo and post-implantation embryo proper. Their ectopic expression of is believed to allow the establishment of an embryonic-like transcriptional cascade that initiates and propagates an otherwise dormant endogenous core pluripotency program within a host cell. Certain other reprogramming determinants, such as Tert, Klf-4, c-Myc, SV40 Large T Antigen ("SV40LT") and short hairpin RNAs targeting p53 ("shRNA-p53") have been applied. There determinants may not be potency-determining factors in and of themselves, but have been reported to provide advantages in reprogramming. For example, TERT and SV40LT are understood to enhance cell proliferation to promote survival during reprogramming, while others such as short hairpin targeting of p53 inhibit or eliminate reprogramming barriers, such as senescence and apoptosis mechanisms. In each case, an increase in both the speed and efficiency of reprogramming is observed. In addition, microRNAs ("miRNAs") are also known to influence pluripotency and reprogramming, and some miRNAs from the miR-290 cluster have been applied in reprogramming studies. For example, the introduction of miR-291-3p, miR-294 or miR-295 into fibroblasts, along with pluripotency-related genes, has also been reported to increase reprogramming efficiency.

While various vectors and reprogramming factors in the art appear to present multiple ingredients capable of establishing reprogramming in cells, a high degree of complexity occurs when taking into account the stoichiometric expression levels necessary for successful reprogramming to take hold. For example, somatic cell reprogramming efficiency is reportedly fourfold higher when OCT-4 and SOX2 are encoded in a single transcript on a single vector in a 1:1 ratio, in contrast to delivering the two factors on separate vectors. The latter case results in a less controlled uptake ratio of the two factors, providing a negative impact on reprogramming efficiency. One approach towards addressing these obstacles is the use of polycistronic vectors, such as inclusion of an internal ribosome entry site ("IRES"), provided upstream of transgene(s) that is distal from the transcriptional promoter. This organization allows one or more transgenes to be provided in a single reprogramming vector, and various inducible or constitutive promoters can be combined together as an expression cassette to impart a more granular level of transcriptional control for the plurality of transgenes. These more specific levels of control can benefit the reprogramming process considerably, and separate expression cassettes on a vector can be designed accordingly as under the control of separate promoters.

Although there are advantages to providing such factors via a single, or small number of vectors, upper size limitations on eventual vector size do exist, which can stymie attempts to promote their delivery in a host target cell. For example, early reports on the use of polycistronic vectors were notable for extremely poor efficiency of reprogramming, sometimes occurring in less than 1% of cells, more typically less than 0.1%. These obstacles are due, in-part, to certain target host cells possessing poor tolerance for large constructs (e.g., fibroblasts), or inefficient processing of IRES sites by the host cells. Moreover, positioning of a factor in a vector expression cassette affects both its stoichiometric and temporal expression, providing an additional variable impacting reprogramming efficiency. Thus, some improved techniques can rely on multiple vectors each encoding one or more reprogramming factors in various expression cassettes. Under these designs, alteration of the amount of a particular vector for delivery provides a coarse, but relatively straightforward route for adjusting expression levels in a target cell.

Finally, in some instances, there may be further benefits in altering the chemical and/or atmospheric conditions under which reprogramming will take place. For example, as the pre-implantation embryo is not vascularized and hypoxic (similar to bone marrow stem-cell niches) reprogramming under hypoxic conditions of 5% O₂, instead of the atmospheric 21% O2, may further provide an opportunity to increase the reprogramming efficiency. Similarly, chemical induction techniques have been used in combination with reprogramming, particularly histone deacetylase (HDAC) inhibitor molecule, valproic acid (VPA), which has been found wide use in different reprogramming studies. At the same time, other small molecules such as MAPK kinase (MEK)-ERK ("MEK") inhibitor PD0325901, transforming growth factor beta ("TGF-β") type I receptor ALK4, ALK5 and ALK7 inhibitor SB431542 and the glycogen synthase kinase-3 ("GSK3") inhibitor CHIR99021 have been applied for activation of differentiation-inducing pathways (e.g. BMP signaling), coupled with the modulation of other pathways (e.g. inhibition of the MAPK kinase (MEK)-ERK pathway) in order to sustain self-renewal. Other small molecules, such as Rho-associated coiled-coil-containing protein kinase ("ROCK") inhibitors, such as Y-27632 and thiazovivin ("Tzv") have been applied in order to promote survival and reduce vulnerability of pSCs to cell death, particularly upon single-cell dissociation.

In addition to the choice of delivery vectors, reprogramming factor combinations, and conditions for reprogramming, further variations must consider the nature of the host target cell for reprogramming. To date, a wide variety of cells have served as sources for reprogramming including fibroblasts, stomach and liver cell cultures, human keratinocytes, adipose cells, and frozen human monocyte. Clearly, there is a wide and robust potential for dedifferentiation across many tissues sources. Nevertheless, it is widely understood that depending on the donor cell type, reprogramming is achieved with different efficiencies and kinetics. For example, although fibroblasts remain the most popular donor cell type for reprogramming studies, other types of cells such as human primary keratinocytes transduced with Oct-4, Sox-2, Klf-4 and c-Myc have been reported to reprogram 100 times more efficiently and two-fold faster. Additionally, some other cell types, such as cord blood cells, may only require a subset of reprogramming factors, such as Oct-4 and Sox-2 for dedifferentiation to take hold, while neural progenitor cells may only require Oct-4. Without being bound to any particular theory, it is believed that differences in reprogramming efficiency and/or reprogramming factor requirements of specific host cells result from high endogenous levels of certain reprogramming factors and/or intrinsic epigenetic states that are more amenable to reprogramming.

Although these many other sources have been used across studies for the generation of iPSCs, mononuclear cells (MNCs) from peripheral blood (PB) are a highly attractive host cell candidate due to convenience and features as an almost unlimited resource for cell reprogramming. Of the various cell types present in peripheral blood, B-cells represent a large percentage of the blood mononuclear cell population, as possess particularly unique features well-suited for reprogramming application.

First, PB cells in general are relatively easy to isolate (e.g., blood draw) compared to isolation from other sources such as fibroblasts (e.g., skin biopsy). Second, such cells do not require laborious culturing and propagation prior to reprogramming, thereby reducing the overall time from which reprogramming iPSCs can be obtained. Third, B-cells in particular possess desirable properties related to both plasticity and subsequent culturing as cell lines. For example, it is widely understood that the reprogramming process benefits from a degree of inherent physiologic plasticity within the target host cell. Because B-cells already possess important physiological roles as immune cell precursors, and a measure of transdifferentiation capacity into hematopoietic precursor cells for blood creation, a high degree of cellular plasticity is present that is well-suited for reprogramming applications. Fourth, B-cells are readily transformed *in vitro* by Epstein-Barr virus ("EBV") to generate lymphoblastoid cell ("LCs") cell lines. This existing experience with LC cell lines has already long served as a resource for immunologic, epidemiologic, and rare disease studies. Fifth, facilities capable of managing collections of LCs and cell lines for storage and distribution already exist. Previously frozen LCs and cell line collections stored worldwide can therefore serve as an existing resource for generating LCL-iPSCs, as well as establishing new opportunities to collect LCs from living donors. Thus, receptivity of LCs to transformation and subsequent propagation provides a further benefit of renewable source material.

Following successful reprogramming, clonal selection allows for generation of pluripotent stem cell lines. Ideally, such cells possess requisite morphology (i.e., compact colony, high nucleus to cytoplasm ratio and prominent nucleolus), self-renewal capacity for unlimited propagation in culture (i.e., immortal), and with the capability to differentiate into all three germ layers (e.g., endoderm, mesoderm and ectoderm). Further techniques to characterize the pluripotency of a given population of cells include injection into an immunocompromised animal, such as a severe combined immunodeficient ("SCID") mouse, for formation of teratomas containing cells or tissues characteristic of all three germ layers.

Described herein is a composition of lymphoblastoid B-cell derived induced pluripotent stem cells ("LCL-iPSCs"). As disclosed herein, the composition of B-cell derived induced pluripotent stem cells includes cells generated by providing a quantity of lymphoid cells (LCs), delivering a quantity of reprogramming factors into the LCs, culturing the LCs in a reprogramming media for at least 7 days, and further culturing the LCs in an induction media for at least 10 days, wherein delivering the reprogramming factors, culturing and further culturing generates the lymphoid-cell derived induced pluripotent stem cells. As described herein , the reprogramming factors may be Oct-4, Sox-2, Klf-4, c-Myc, Lin-28, SV40 Large T Antigen ("SV40LT"), and short hairpin RNAs targeting p53 ("shRNA-p53"). As described herein , these reprogramming factors may be encoded in a combination of vectors including pEP4 E02S ET2K, pCXLE-hOCT3/4-shp53-F, pCXLE-hSK, and pCXLE-hUL. As described herein , the reprogramming media may include PD0325901, CHIR99021, HA-100, and A-83-01. As described herein , the culturing the LCs in a reprogramming media may be for 8-14 days and further culturing the LCs in an induction media may be for 1-12 days.

Reprogramming factors can also include one or more of following: Oct-4, Sox-2, Klf-4, c-Myc, Lin-28, SV40LT, shRNA-p53, nanog, Sall4, Fbx-15, Utf-1, Tert, or a Mir-290 cluster microRNA such as miR-291-3p, miR-294 or miR-295. Also disclosed herein, the reprogramming factors may be encoded by a vector. Also disclosed herein, the vector can be, for example, a non-integrating episomal vector, minicircle vector, plasmid, retrovirus (integrating and non-integrating) and/or other genetic elements known to one of ordinary skill. As described herein , the reprogramming factors may be encoded by one or more oriP/EBNA1 derived vectors. Also disclosed herein, the vector may encode one or more reprogramming factors, and combinations of vectors can be used together to deliver one or more of Oct-4, Sox-2, Klf-4, c-Myc, Lin-28, SV40LT, shRNA-p53, nanog, Sall4, Fbx-15, Utf-1, Tert, or a Mir-290 cluster microRNA such as miR-291 -3p, miR-294 or miR-295. For example, oriP/EBNA1 is an episomal vector that can encode a vector combination of multiple reprogramming factors, such as pCXLE-hUL, pCXLE-hSK, pCXLE-hOCT3/4-shp53-F, and pEP4 EO2S T2K.

The reprogramming factors may be delivered by techniques known in the art, such as nuclefection, transfection, transduction, electrofusion, electroporation, microinjection, cell fusion, among others. Also disclosed herein, the reprogramming factors may be provided as RNA, linear DNA, peptides or proteins, or a cellular extract of a pluripotent stem cell.

Also disclosed herein, the reprogramming media may comprise at least one small chemical induction molecule. The at least one small chemical induction molecule may comprise PD0325901, CHIR99021, HA-100, A-83-01., valproic acid (VPA), SB431542, Y-27632 or thiazovivin ("Tzv"). As disclosed herein, culturing the LCs in a reprogramming media may be for at least 7, 8, 9, 10, 11, 12, 13, 14, 15, or 16 days. As disclosed herein, culturing the LCs in a reprogramming media may be for at least 7, 8, 9, 10, 11, 12, 13, 14, 15, or 16 days. As disclosed herein, culturing the LCs in an induction media may be for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days.

Also disclosed herein, the LCL-iPSCs may be derived from lymphoblastoid B-cells previously isolated from a subject, by for, example, drawing a blood sample from the subject. Also disclosed herein, the LCs may be isolated from a subject possessing a disease mutation. For example, subjects possessing any number of mutations, such as autosomal dominant, recessive, sex-linked, can serve as a source of LCs to generate LCL-iPSCs possessing said mutation. As described herein , the disease mutation may be associated with a neurodegenerative disease, disorder and/or condition. As described herein , the disease mutation may be associated with an inflammatory bowel disease, disorder, and/or condition.

As described herein , the LCL-iPSCs possess features of pluripotent stem cells. Some exemplary features of pluripotent stem cells including differentiation into cells of all three germ layers (ectoderm, endoderm, mesoderm), either *in vitro* or *in vivo* when injected into an immunodeficient animal, expression of pluripotency markers such as Oct-4, Sox-2, nanog, TRA-1-60, TRA-1-81, SSEA4, high levels of alkaline phosphatase ("AP") expression, indefinite propagation in culture, among other features recognized and appreciated by one of ordinary skill.

Also disclosed herein, is a pharmaceutical composition including a quantity of lymphoid-cell derived induced pluripotent stem cells generated by the above described methods, and a pharmaceutically acceptable carrier.

Also described herein is an efficient method for generating induced pluripotent stem cells, including providing a quantity of cells, delivering a quantity of reprogramming factors into the cells, culturing the cells in a reprogramming media for at least 7 days, and further culturing the cells in an induction media for at least 10 days, wherein delivering the reprogramming factors, culturing and further culturing generates induced pluripotent stem cells. As disclosed herein, the cells may be primary culture cells. As described herein , the cells may be lymphoblastoid B-cells, further including lymphoblastoid B-cells from a cell line.

As described herein , the reprogramming factors may be Oct-4, Sox-2, Klf-4, c-Myc, Lin-28, SV40 Large T Antigen ("SV40LT"), and short hairpin RNAs targeting p53 ("shRNA-p53"). As described herein , these reprogramming factors may be encoded in a combination of vectors including pEP4 E02S ET2K, pCXLE-hOCT3/4-shp53-F, pCXLE-hSK, and pCXLE-hUL. As described herein , the reprogramming media may include PD0325901, CHIR99021, HA-100, and A-83-01. Also disclosed herein, culturing the cells in a reprogramming media may be for 8-14 days and further culturing the cells in an induction media may be for 1-12 days.

Reprogramming factors can also include one or more of following: Oct-4, Sox-2, Klf-4, c-Myc, Lin-28, SV40LT, shRNA-p53, nanog, Sall4, Fbx-15, Utf-1, Tert, or a Mir-290 cluster microRNA such as miR-291-3p, miR-294 or miR-295. Also disclosed herein, the reprogramming factors may be encoded by a vector. Also disclosed herein, the vector can be, for example, a non-integrating episomal vector, minicircle vector, plasmid, retrovirus (integrating and non-integrating) and/or other genetic elements known to one of ordinary skill. As described herein , the reprogramming factors may be encoded by one or more oriP/EBNA1 derived vectors. As described herein , the vector encodes one or more reprogramming factors, and combinations of vectors can be used together to deliver one or more of Oct-4, Sox-2, Klf-4, c-Myc, Lin-28, SV40LT, shRNA-p53, nanog, Sall4, Fbx-15, Utf-1, Tert, or a Mir-290 cluster microRNA such as miR-291 -3p, miR-294 or miR-295. For example, oriP/EBNA1 is an episomal vector that can encode a vector combination of multiple reprogramming factors, such as pCXLE-hUL, pCXLE-hSK, pCXLE-hOCT3/4-shp53-F, and pEP4 EO2S T2K.

As described herein , the reprogramming factors may be delivered by techniques known in the art, such as nuclefection, transfection, transduction, electrofusion, electroporation, microinjection, cell fusion, among others. Also disclosed herein, the reprogramming factors may be provided as RNA, linear DNA, peptides or proteins, or a cellular extract of a pluripotent stem cell. Also disclosed herein, the cells may be treated with sodium butyrate prior to delivery of the reprogramming factors. Also disclosed herein, the cells may be incubated or 1, 2, 3, 4, or more days on a tissue culture surface before further culturing. This can include, for example, incubation on a Matrigel coated tissue culture surface. Also disclosed herein, the reprogramming conditions include application of norm-oxygen conditions, such as 5% O₂, which is less than atmospheric 21% O₂.

Also disclosed herein, the reprogramming media may comprise at least one small chemical induction molecule. As described herein , the at least one small chemical induction molecule may comprise PD0325901, CHIR99021, HA-100, A-83-01, valproic acid (VPA), SB431542, Y-27632 or thiazovivin ("Tzv"). As described herein , the induction media may be a chemically defined, serum-free media. This disclosure includes, for example, mTeSR1 and mTeSR2 media. As described herein , culturing the cells in a reprogramming media may be for at least 7, 8, 9, 10, 11, 12, 13, 14, 15, or 16 days. Also disclosed herein, the reprogramming media may be added without aspirating previously seeded media, or replaced following aspiration of previously seeded media. Reprogramming media may be added or replaced daily, or on alternating days. As described herein , culturing the cells in a reprogramming media may be for at least 7, 8, 9, 10, 11, 12, 13, 14, 15, or 16 days. Also disclosed herein, culturing the cells in an induction media may be for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. Also disclosed herein, culturing the cells in induction media may include a combination of induction media and reprogramming media, wherein increasingly higher amounts of induction media are added in specific ratios to an amount of reprogramming media (e.g., 1:1, 2:1, 3:1 ratios of induction media to reprogramming media).

Efficiency of reprogramming is readily ascertained by one of many techniques readily understood by one of ordinary skill. For example, efficiency can be described by the ratio between the number of donor cells receiving the full set of reprogramming factors and the number of reprogrammed colonies generated. Measuring the number donor cells receiving reprogramming factors can be measured directly, when a reporter gene such as GFP is included in a vector encoding a reprogramming factor. Alternatively, indirect measurement of delivery efficiency can be provided by transfecting a vector encoding a reporter gene as a proxy to gauge delivery efficiency in paired samples delivering reprogramming factor vectors. Further, the number of reprogrammed colonies generated can be measured by, for example, observing the appearance of one or more embryonic stem cell-like pluripotency characteristics such as alkaline phosphatase (AP)-positive clones, colonies with endogenous expression of transcription factors OCT4 or NANOG, or antibody staining of surface markers such as Tra-1-60. In another example, efficiency can be described by the kinetics of induced pluripotent stem cell generation.

### Example 1

### General iPSC Reprogramming Protocol for Lymphoblastoid Cell Line

Generally, an improved method for reprogramming can be described as first involving nuclection of a target host cell with a combination of plasmids, followed by 2 days of incubation, daily addition of reprogramming media (without aspiration of old media) on each of days 3-5, replacement of reprogramming media (with aspiration) on day 6, daily addition of reprogramming media (without aspiration of old media) on each of days 7-9, replacement of reprogramming media (with aspiration) on day 10, alternate daily addition of reprogramming media (without aspiration of old media) on days 10-16, Small colonies may appear as early as day 11, with substantial numbers of colonies becoming visible by day 17. Media switching into progressively increasing amounts of serum-free, complete media, mTeSR1 is provided on days 18-20. By day 24, reprogrammed colonies are readily apparent, and can be antibody stained for live cell imaging for confirmation. Throughout days 25-29, additional colonies can be isolated for sub-cloning. By day 30, previously isolated colonies begin to adhere, display normal iPSC morphology and can be stored or subsequently serially passaged as cell lines. Using the described techniques the inventors can achieved at least 10% conversion efficiency, representing at least 3-8 fold improvement compared to existing reprogramming studies.

### Example 2

### Lymphoblastoid cell line to iPSC Reprogramming Protocol: Nucleofection

Reprogramming of LC cell lines can be achieved through the following exemplary method. On the first day, 3x10⁶ cells are harvested from a flask of LCs triturated in a 15 ml conical tube, and washed 1x with PBS. Cell are then centrifuged at 200x g, added to 1 ml PBS added and transfer to microfuge tube. Further centrifugation at 200x g, is followed by careful aspiration of PBS carefully, and observation of a small cell pellet.

For delivery of reprogramming vector, a nucleofector device, such as Lonza/Amaxa, can be used. For nucleofection, 2 microfuge tubes per reaction are prepared with Nucleofector solution (82 µl) + supplement (18 µl) in a first tube, per manufacturer protocol (Cell Line Nucleofector Kit C VACA-1004). In the second tube, a plasmid mixture is prepared with 1.5 µg of each of the following plasmid DNA: pEP4 E02S ET2K: (Addgene Plasmid No. 20927), pCXLE-hOCT3/4-shp53-F: (Addgene Plasmid No. 27077), pCXLE-hSK: (Addgene Plasmid No. 27078), pCXLE-hUL: (Addgene Plasmid No. 27080).

Following plasmid mixture preparation, a 15ml conical and a Matrigel coated 6-well plate are prepared. In the 15 ml conical tube, 3ml pre-warmed LC media (RPMI1640+ 15%FBS +2mM L-glutamine) + 0.5% PSA is added. In the 6-well plate, 3/6 wells coated with growth factor-reduced Matrigel (BD#354230). A minimum of 1 hour of thin-film coating is required at room temperature. 0.5mg of Matrigel is sufficient to coat an entire 6-well plate. Add 1 ml LC media to each well and keep ready for cells post-nucleofection.

After preparation of cell culture tubes and plates, the plasmid mixture is added to the microfuge tube. Flicking of tube for mixing is preferable, or pipetting can be performed, but carefully and not more than 1-2 times. The cell and plasmid mixture is gently transferred to nucleofection cuvette provided with the kit, with careful handling necessary to avoid of bubbles.

The cuvette is placed in in the Nucleofector device, with application of program E-010, and removal of cuvette after nucleofection program is complete. The nucleofected cell and plasmid mixture are gently transferred using plastic transfer pipet provided with the kit to the previously prepared 15 ml conical containing 3 ml of LC media. Wash the cuvette once with 0.1 ml media and transfer to 15 ml conical. Cells are divided gently and evenly (1ml/well) into 3 wells of Matrigel coated plate containing 1ml/well of LC media. Place gently incubator at 37°C and 20% O₂. Plate is incubated and to be left undisturbed for over the next 2 days.

### Example 3

### Lymphoblastoid cell line to iPSC Reprogramming Protocol: Induction of Stem Cell Pluripotency

On the third day, 1ml/well of reprogramming media is added to existing media in the well each, and repeated the following fourth and fifth days. Old media and cells are not aspirated when fresh media is added. Instead, the fresh reprogramming media ("RM") is added to the existing media from previous days.

On the sixth day, the media and dead cells are gently aspirated, and replace with 2ml/well of fresh RM. For the next three days (i.e., seventh, eighth, ninth days), 1 ml of RM for next 3 days is added. Again, old media and cells are not aspirated when fresh media is added. Instead, the fresh RM media is added to the existing media from previous days.

On the tenth day, the media and dead cells are gently aspirated, and replace with 2ml/well of fresh RM on alternate days (i.e., tenth, twelfth, fourteenth, and sixteenth days)

Small colonies may begin appearing as early the eleventh day. Beginning on approximately the seventeenth day, media is progressively altered to increasing amounts of serum-free, complete media, mTeSR1. On the seventeenth day, media is switched to a 1:1 ratio of mTeSR1 (#05850/05896) and RM. On the eighteenth day, media is switched to a 2:1 ratio of mTeSR1 (#05850/05896) and RM. On the nineteenth day, media is switched to a 3:1 ratio of mTeSR1 (#05850/05896) and RM. On the twentieth day, media is switched to a 100% mTeSR1 (#05850/05896) and RM.

### Example 4

### Lymphoblastoid cell line to iPSC Reprogramming Protocol: Colony Isolation, Expansion and Passaging

On the twenty-first day, a high amount of cell death is observed. By the twenty-fourth day, reprogrammed colonies become readily apparent throughout the plated cells, and a live cell stain in 1 well of 3/6 plate can be performed using Tra-1-60 DyLight 488 antibody (Stemgent #09-0068). Positive colonies can be sub-cloned into a new plate of 1/12 GFR Matrigel coated plates. Sub-cloning is performed by cutting colonies with fire-polished glass Pasteur pipets and transferring the cut floating chunks to a new well of a 12-well plate using a p200 pipette.

On the twenty-fifth days up to the twenty-ninth day, additional colonies can be isolated, as picked from remaining wells and transferred to 1/12 wells per clone. Usually a TRA-1-60 live cell stain is required to identify reprogrammed colonies during days 25-27.

By the twenty-eight or twenty-ninth days, colonies in the original 3/6 wells have defined edges and may be isolated by an experienced PSC researcher solely on morphology, without a TRA-1-60 live cell stain.

On the thirtieth day, previously isolated colonies begin to adhere and display to show normal iPSC morphology with slight differentiation. An additional culturing period, up to approximately 10 more days may be necessary for a new iPSC clone to establish and expand before passaging can be performed. Early clonal lines are passaged manually using a p1000 tip for the first two passages - from 1/12 well to 2/12 wells and then 1/6 well. Cells capable of expansion and passaging can subsequently be stored for banking, cryopreservation and characterization of the clonal iPSC lines.

### Example 5

### Sample Reprogramming Media (RM) for use in Reprogramming Protocols

The following reprogramming media can be used in reprogramming techniques described in the application:
- DMEM/F12
- 100ng/ml bFGF (Peprotech)
- 1% NEAA
- 1:1000 (∼1000 units) hLIF (Millipore #LIF1010)
- 1% GlutaMax
- 0.5µM PD0325901 (Cayman #13034)
- 1% N2
- 3µM CHIR99021 (Tocris #4423)
- 2% B27
- 10µM HA-100 (Santa Cruz #203072)
- 0.5% Antibiotic-Antimycotic (Gibco #15240-062)
- 0.5µM A-83-01 (Tocris #2939)
- 0.1µM β-mercaptoethanol

### Example 6

### General iPSC Reprogramming Protocol for Cultures of Primary Cells

The improved methods described herein are readily extendible to other types of cells, including primary cell cultures. For example, primary culture cells can be cultured in a T-75 flask until reaching approximately 90% confluence. Reprogramming of the primary culture cells can be achieved by plasmid nucleofection, performed using oriP/EBNA1 based pCXLE-hUL, pCXLE-hSK, pCXLE-hOCT3/4-shp53-F, and pEP4 EO2S T2K plasmid vectors (Addgene). As described, a nucleofector device, such as Lonza/Amaxa, can be used per manufacturer protocol. Briefly, primary culture cells (1 x 10⁶ cells per nucleofection) are harvested and centrifuged at 200x g for 5 minutes. The cell pellet is re-suspended carefully in Nucleofector Solution (VPD-1001, Lonza) and combined with episomal plasmids (1.5 µg per plasmid) expressing Oct-4, Sox-2, Klf-4, c-Myc, Lin-28, SV40LT and p53 shRNA. The cell/DNA suspension is transferred into the Nucleofector and the E-010 program applied.

### Example 7

### Induction of Stem Cell Pluripotency with Small Molecule Induction

Immediately after nucleofection, cells were plated on BD Matrigel coated dishes and fed with MEBM. All cultures were be maintained under norm-oxygen conditions (5% O₂) during reprogramming, which further enhances the efficiency of iPS cell generation. The media was kept on for 48h and gradually changed to reprogramming media consisting of DMEM/F12, 1% Glutamax, 1% NEAA, 1% N2, 2% B27, 1% antibiotic-antifungal, 0.1 mM beta-mercaptoethanol, 100 ng/mL basic fibroblast growth factor ("Bfgf"), and 1000 units/mL human Leukemia Inhibitory Factor ("hLIF"). In addition, small molecules were supplemented in the RM to enhance reprogramming efficiency. The small molecules used were, 1) HA-100 (10 µM), 2) glycogen synthase kinase 3β inhibitor of the Wnt/β-catenin signaling pathway (CHIR99021, 3 µM), 3) MEK pathway inhibitor (PD 0325901, 0.5 µM), 4) Selective inhibitor of TGF-β type I receptor ALK5 kinase, type I activin/nodal receptor ALK4 and type I nodal receptor ALK7 (A 83-01, 0.5 µM). Fresh RM was added daily to the conditioned media. This was repeated daily for the next 4 days. On the 7th day post nucleofection, all medium was aspirated from the wells and cells were fed with RM. Media was changed every 3rd day to fresh RM for the next 13 days (day 20 post nucleofection).

### Example 8

### Generation of iPSC Colonies

Colonies with ES/iPSC-like morphology appeared at day 25-31 post-nucleofection. Subsequently, colonies with the best morphology were picked on day 31 and transferred to BD Matrigel™ Matrix for feeder-independent maintenance of hiPSCs in chemically-defined mTeSR1 medium.

### Example 9

### Generation of human LCL-iPSCs using Episomal Plasmids

The representative LCLs primarily reported in this study were derived from a control (GM22649) and SMA (GM10684) patient. They were maintained in RPMI 1640 (Life Technologies) media supplemented with 15% FBS and 2 mM L-glutamine, and cultured at 37°C and 5% CO2 in a humidified incubator. Upon iPSC generation at CSMC, they were renamed 49iCTR-n2, 49iCTRn6, 84iSMA-n4, and 84iSMA-n12 to reflect catalog number, control line and clone number. LCLs were reprogrammed into virus-free iPSC lines with either the Cell Line Nucleofector Kit C (VACA-1004, Lonza) or B-cell Nucleofector Kit (VPA-1001, Lonza) using 1.5 µg of each episomal plasmid (Addgene) expressing 7 factors: OCT4, SOX2, KLF4, L-MYC, LIN28, SV40LT and p53 shRNA (pEP4 E02S ET2K, pCXLE-hOCT3/4-shp53-F, pCXLE-hUL, and pCXLE-hSK). This method has a significant advantage over viral transduction, because exogenously introduced genes do not integrate and are instead expressed episomally in a transient fashion. LCLs (1 x 106 cells per nucleofection) were harvested, centrifuged at 1500rpm for 5 minutes, resuspended carefully in Nucleofector® Solution and the E-010 program was applied. These nucleofected cells were plated on feeder-independent BD Matrigel™ growth factor-reduced Matrix (Corning/BD Biosciences, #354230). All cultures were maintained at 20% O2 during the reprogramming process. Cells were initially cultured in the original LCL medium for 3 days postnucleofection and gradually transitioned to reprogramming media (RM) by adding 1 ml RM to the original LCL media daily for the next 3 days to aid in LCL attachment. Reprogramming media contains DMEM/F12, 1% NEAA, 1% GlutaMax, 1% N2, 2% B27, 0.5% AntibioticAntimycotic (Gibco #15240-062), 0.1µM β-mercaptoethanol, 100ng/ml bFGF (Peprotech), 1:1000 (∼1000 units) hLIF (Millipore, #LIF1010), 0.5µM PD0325901 (Cayman Chemicals, #13034), 3µM CHIR99021 (Tocris, #4423), 10µM HA-100 (Santa Cruz Biotech, #203072), and 0.5µM A-83-01 (Tocris, #2939). The cells were maintained in RM for next 15 days with fresh media replenishment every other day. They were then gradually changed to chemically-defined mTeSR®1 medium between 17-20 days post-nucleofection. Individual LCL-iPSC colonies with ES/iPSC-like morphology appeared between day 25-32 and those with best morphology were mechanically isolated, transferred onto 12-well plates with fresh Matrigel™ Matrix, and maintained in mTeSR®1 medium. The iPSC clones were further expanded and scaled up for further analysis.

### Example 10

### Alkaline Phosphatase Staining

Alkaline Phosphatase staining was performed using the Alkaline Phosphatase Staining Kit II (Stemgent, Cat no. 00-0055) according to the manufacturer's instructions.

### Example 11

### Immunohisto/cytochemistry

LCL-iPSCs or differentiated cells were plated on glass coverslips or optical-bottom 96-well plates (Thermo, # 165305) and subsequently fixed in 4% paraformaldehyde. Intestinal organoids were fixed in 4% paraformaldehyde, transferred to 30% sucrose, embedded in

HistoPrep (Thermo Fisher Scientific) and cut into 20 µm sections. All cells were blocked in 510% goat or donkey serum with 0.1% Triton X-100 and incubated with primary antibodies either for either 3hrs at room temperature or overnight at 4oC. Cells were then rinsed and incubated in species-specific AF488 or AF594-conjugated secondary antibodies followed by Hoechst 33258 (0.5 µg/ml; Sigma) to counterstain nuclei. Cells were imaged using Nikon/Lecia microscopes.

Antibodies used for immunocytochemistry and immunoblotting include: (as listed by antigen, dilution, catalog no., isotype, and manufacturer) SSEA4, 1:250, MAB4304, mIgG3, Millipore; TRA-1-60, 1:250, 09-0010, IgM, Stemgent; TRA-1-81, 1:250,09-0011, mIgM, Stemgent; OCT4, 1:250, 09-0023, Rabbit IgG, Stemgent; NANOG, 1:250, 09-0020, Rabbit IgG, Stemgent; SOX2,1:500, AB5603, Rabbit IgG, Millipore; TuJI (β3-tubulin), 1:1000, T8535, mIgG2b, Sigma; CDX2, 1:500, NBP1-40553, IgG, Novus; FABP2, 1:500, AF3078, IgG, R & D systems; Collagen Type 1, 1:500, 600-401-103-0.1, Rabbit Rockland; CD73, 1:500, 550257, mIgG1, BD Pharmingen; NKX6.1, 1:100 F55A10, mIgG1, DSHB Iowa; HB9, 1:25, 81.5C10, mIgG1, DSHB Iowa; ISELT1, 1:250, AF1837, Goat IgG, R & D systems; SMI32, 1:1000, SMI-32R, mIgG1, Covance; CHAT, 1:250, AB144P, Goat IgG, Millipore; SMN, 1:250, 610647, mIgG1, BD Biosciences; Cox-IV, 1:1000, 4850s, Rabbit Cell signaling; GAPDH, 1:1000, ab9484, mIgG2b, Abcam.

### Example 12

### Flow Cytometry

LCL-iPSCs were dissociated into a single cell suspension using Accutase (Millipore, #SCR005). Surface staining of IPSCs was carried out using SSEA4 (R&D Systems, FAB1435A).

Cells were then fixed, permeabilized and stained intracellularly for Oct3/4 (BD Pharmingen, 560186). Recommended isotypes were used according to the antibodies recommendation (R&D Systems, FABIC007 and BD Pharmingen, 562547). All samples were analyzed using a BD LSRFortessa flow cytometer using BD FACSDiva software. All images were generated using FloJo software.

### Example 13

### Karyotype

Human LCL-iPSCs were incubated in Colcemid (100 ng/mL; Life Technologies) for 30 minutes at 37°C and then dissociated using TrypLE for 10 minutes. They were then washed in phosphate buffered saline (PBS) and incubated at 37°C in 5mL hypotonic solution (1g KCl, 1g Na Citrate in 400mL water) for 30 minutes. The cells were centrifuged for 2.5 minutes at 1500 RPM and resuspended in fixative (methanol: acetic acid, 3:1) at room temperature for 5 minutes. This was repeated twice, and finally cells were resuspended in 500 µl of fixative solution and submitted to the Cedars-Sinai Clinical Cytogenetics Core for G-Band karyotyping.

### Example 14

### PluriTest

Total RNA was isolated using the RNeasy Mini Kit (Qiagen) and subsequently run on a Human HT-12 v4 Expression BeadChip Kit (Illumina). The raw data file (idat file) was subsequently uploaded onto the Pluritest widget online (www.pluritest.org).

### Example 15

### Quantitative RT-PCR

Total RNA was isolated using the RNeasy Mini Kit (Qiagen), and 1 ug of RNA was used to make cDNA using the transcription system (Promega). qRT-PCR was performed using specific primer sequences (Table 2) under standard conditions. "CDS" indicates that primers designed for the coding sequence measured expression of the total endogenous gene expression only, whereas "Pla" indicates that primers designed for the plasmid transgene expression only. Data are represented as mean ± SEM

**Table 2. qRT-PCR Primer Sequences**

| **Gene Name** | **Forward Primer** | **Reverse Primer** |
|---|---|---|
| OCT3/4 CDS | ccccagggccccattttggtacc (SEQ ID NO: 1) | acctcagtttgaatgcatgggagagc (SEQ ID NO: 2) |
| OCT3/4 Pla | cattcaaactgaggtaaggg (SEQ ID NO: 3) | tagcgtaaaaggagcaacatag (SEQ ID NO: 4) |
| SOX2 CDS | ttcacatgtcccagcactaccaga (SEQ ID NO: 5) | tcacatgtgtgagaggggcagtgtgc (SEQ ID NO: 6) |
| SOX2 Pla | ttcacatgtcccagcactaccaga (SEQ ID NO: 7) | tttgtttgacaggagcgacaat (SEQ ID NO: 8) |
| KLF4 CDS | acccatccttcctgcccgatcaga (SEQ ID NO: 9) | ttggtaatggagcggcgggacttg (SEQ ID NO: 10) |
| KLF4 Pla | ccacctcgccttacacatgaaga (SEQ ID NO: 11) | tagcgtaaaaggagcaacatag (SEQ ID NO: 12) |
| LMYC CDS | gcgaacccaagacccaggcctgctcc (SEQ ID NO: 13) | cagggggtctgctcgcaccgtgatg (SEQ ID NO: 14) |
| LMYC Pla | ggctgagaagaggatggctac (SEQ ID NO: 15) | tttgtttgacaggagcgacaat (SEQ ID NO: 16) |
| LIN28 CDS | agccatatggtagcctcatgtccgc (SEQ ID NO: 17) | tcaattctgtgcctccgggagcagggtagg (SEQ ID NO: 18) |
| LIN28 Pla | agccatatggtagcctcatgtccgc (SEQ ID NO: 19) | tagcgtaaaaggagcaacatag (SEQ ID NO: 20) |
| RPL13A | cctggaggagaagaggaaaga (SEQ ID NO: 21) | ttgaggacctctgtgtatttg (SEQ ID NO: 22) |
| B2M | tgctgtctccatgtttgatgt (SEQ ID NO: 23) | tctctgctccccacctctaag (SEQ ID NO: 24) |
| EBNA1 | atcagggccaagacatagaga (SEQ ID NO: 25) | gccaatgcaacttggacgtt (SEQ ID NO: 26) |
| EBNA 2 | catagaagaagaagaggatgaaga (SEQ ID NO: 27) | gtagggattcgagggaattactga (SEQ ID NO: 28) |
| LMP1 | atggaacacgaccttgaga (SEQ ID NO: 29) | tgagcaggatgaggtctagg (SEQ ID NO: 30) |
| BZLF1 | cacctcaacctggagacaat (SEQ ID NO: 31) | tgaagcaggcgtggtttcaa (SEQ ID NO: 32) |
| OriP | tcgggggtgttagagacaac (SEQ ID NO: 33) | ttccacgagggtagtgaacc (SEQ ID NO: 34) |
| GAPDH | accacagtccatgccatcac (SEQ ID NO: 35) | tccaccaccctgttgctgta (SEQ ID NO: 36) |
| TDGF | tccttctacggacggaactg (SEQ ID NO: 37) | agaaatgcctgaggaaagca (SEQ ID NO: 38) |
| NCAM1 | gattcctcctccaccctcac (SEQ ID NO: 39) | caatattctgcctggcctggatg (SEQ ID NO: 40) |
| HAND1 | ccacacccactcagagccatt (SEQ ID NO: 41) | caccccaccaccaaaacctt (SEQ ID NO: 42) |
| MSX1 | cgagaggaccccgtggatgcagag (SEQ ID NO: 43) | ggcggccatcttcagcttctccag (SEQ ID NO: 44) |
| AFP | gaatgctgcaaactgaccacgctggaac (SEQ ID NO: 45) | tggcattcaagagggttttcagtctgga (SEQ ID NO: 46) |
| SMN PCR-RFLP | ctatcatgctggctgcct (SEQ ID NO: 47) | ctacaacacccttctcacag (SEQ ID NO: 48) |

### Example 16

### B-cell Immunoglobulin Heavy (IgH) Chain Rearrangement Assay

Genomic DNA (350ng) was harvested from all cell lines using the MasterPure DNA Purification Kit (Epicenter Biotechnologies). An embryonic stem cell line (H9) was used as a negative control. Primer sets that recognize the three framework regions in the heavy chain locus of the IgH gene were obtained from InVivoScribe Technologies (Cat no.11010010, San Diego, CA) and the PCR was carried out as per the manufacturer's protocol.

### Example 17

### EBV Related Gene Analysis

Genomic DNA (400ng) was harvested from all cell lines and an embryonic stem cell line (H9) was used a negative control. Primers that recognize EBNA-1, EBNA-2, BZLF1, LMP1, OriP, along with GAPDH, which was used as a housekeeping gene, were included in this study (Table 2). PCR was run for 35 cycles at 95°C for 30 sec, 60°C for 30 s, and 72°C for 30s PCR-restriction fragment length polymorphism ("PCR-RFLP") assay

Genomic DNA (200ng) was obtained from all iPSC lines. Primers that recognize exon 8 of the SMA gene (Table 2) were used under the following conditions; 30 cycles at 98°C for 10 sec, 60°C for 30 s, and 72°C for 1min. The PCR product was subsequently digested using the restriction enzyme, Dde1, for 1 hr at 37°C. The digested and undigested PCR products were then visualized after electrophoresis using a 2% agarose gel.

### Example 18

### Neural and Motor Neuron Differentiation

The iPSCs were grown to near confluence under normal maintenance conditions before the start of the differentiation. IPSCs were then gently lifted by Accustase treatment for 5 min at 37°C. 1.5-2.5 X 104 cells were subsequently placed in each well of a 384 well plate in defined neural differentiation medium (SaND) composed of Iscove's modified Dulbecco's medium supplemented with B27-vitamin A (2%) and N2 (1%) with the addition of 0.2uM LDN193189 and 10uM SB431542. After 2 days, neural aggregates were transferred to low adherence PolyHema coated plates. After 6 days, neural aggregates were plated onto laminin-coated 6 well plates to induce rosette formation. From day 12-18, the media was changed to SaND supplemented with 0.1uM retinoic acid and 1uM puromorphin (SaND-TRAP) along with 20ng/ml BDNF, 200ng/ml ascorbic acid, 20ng/ml GDNF and 1mM dbcAMP and neural rosettes were selected using rosette selection media (Stemcell, 05832). The purified rosettes were subsequently cultured in SaND-TRAP supplemented with 100ng of EGF and FGF. These neural aggregates were expanded over a 2 - 7 week period, disassociated with accustase and then plated onto laminin-coated plates. These MN prescursors were then cultured in MN maturation stage 1 media consisting of Dulbecco's modified Eagle's medium (DMEM)/F12, supplemented with 2% B27, retinoic acid (0.1 µM), puromorphin (1 µM), dibutyryl cyclic adenosine monophosphate (1 µM), ascorbic acid (200 ng/ml), brain-derived neurotrophic factor

(10 ng/ml), and glial cell line-derived neurotrophic factor (10 ng/ml) for 7 days and then cultured in MN maturation stage 2 media consisting of Neurobasal supplemented with 1% N2, ascorbic acid (200 ng/ml), dibutyryl cyclic adenosine monophosphate (1µM), brain-derived neurotrophic factor (10 ng/ml), and glial cell line-derived neurotrophic factor (10 ng/ml).

### Example 19

### Three-Dimensional Intestinal Organoids and Intestinal Epithelial Cells From iPSCs

To induce definitive endoderm formation, all iPSCs were cultured with a high dose of Activin A (100ng/ml, R&D Systems) with increasing concentrations of FBS over time (0%, 0.2% and 2% on days 1, 2 and 3 respectively). Wnt3A (25ng/ml, R&D Systems) was also added on the first day of endoderm differentiation. To induce hindgut formation, cells were cultured in Advanced DMEM/F12 with 2% FBS along with Wnt3A and FGF4 (500ng/ml, R&D Systems). After 3-4 days, free floating epithelial spheres and loosely attached epithelial tubes became visible and were harvested. These epithelial structures were subsequently suspended in Matrigel containing R-Spondin-1, noggin, EGF (500ng/ml, 100ng/ml and 100ng/ml respectively, all R&D

Systems) and then overlaid in intestinal medium containing R-Spondin-1, noggin, EGF (500ng/ml, 100ng/ml and 100ng/ml respectively, all R&D Systems) and B27 (IX, Invitrogen). Organoids were passaged every 7-10 days thereafter.

### Example 20

### Cardiac-EB Differentiation

To direct iPSCs towards beating cardiac mesoderm, floating embryoid bodies (EBs) were formed (20,000 cells/well) from iPSCs by single cell dissociation in sterile 384-well (V-shaped bottom) PCR plates in the presence of 20 ng/ml BMP-4 (Peprotech, USA) in IMDM EB differentiation media containing 17% knock-out serum replacement (KOSR), Matrigel and ROCK inhibitor, and devoid of FGF2. The plates were centrifuged at 200rcf to settle the EBs at the bottom of the V-shaped wells and grown at 37°C and 5% CO2. After 2 days of differentiation, EBs were collected, pooled and cultured in poly-HEMA coated T-25 flasks. At day 4, media was BMP-4 is withdrawn and media changed to cardiac differentiation media (IMDM EB media) supplemented with IWR-1 endo (Cayman chemical, 10 µM final). EBs (∼40 EBs/well) were attached to 0.1% Gelatin coated 96 well optical bottom plates and fresh medium was replaced every other day. After 10 days, the IMDM EB media KOSR content was decreased to 2.5%. EBs were examined daily for beating starting on day 15 and typically beat regularly around 2530 days of differentiation.

### Example 21

### Western Blotting

Cells were collected and homogenized with RIPA buffer (Cell Signaling cat# 9806) with protease and phosphates inhibitor. Protein concentration was then determined with BCA protein assay (Thermo Scientific, # 23225). Equal amounts of Motor neurons and iPSCs protein lysates were heat denatured and separated on an Any kD mini-protean precast gel (Bio-Rad, #4569036). Gel was then transferred on to midi format 0.2uM Nitrocellulose Membrane (Bio-Rad cat#170-4159). Blot was blocked in Odyssey Blocking Buffer (LI-COR Cat # 92740000) for 1 hr and then incubated in primary antibody for SMN (1:1000 BD bioscience cat# 610647) and COXIV (1:1000 Cell Signaling Cat # 4850s) overnight at 4°C. After incubation with infrared conjugated secondary antibodies (Li-cor cat #92668070 and 92632211) for one hour, blots were then washed with TBST and bands were visualized by scanning membrane on Oddesy infrared imaging system (Li-Cor Biosciences).

### Example 22

### Results and Discussions

Reprogramming LCLs to iPSCs using existing episomal nonintegrating protocols provided no identifiable iPSC clones even after 35-40 days. Hence, the Inventors developed a new episomal ("OriP/EBNA 1") plasmid reprogramming method. The optimization included two vital elements: (a) use of seven reprogramming factors, including POU5F1(also known as OCT3/4), SOX2, KLF4, LIN28 (also known as LIN28A), nontransforming L-MYC, SV40 large T antigen ("SV40LT"), and shRNA against p53, in specific stoichiometry to minimize lymphoblastoid cell death and (b) a medium-feeding schedule to promote high surface attachment of the nucleofected LCLs **(****Fig. 1A****).** This novel protocol resulted in successful generation of multiple adherent LCL-iPSC clones that could be mechanically isolated and scaled up for expansion after 27-32 days **(Fig.lA).** Notably, the reprogramming success was very reliable, at 100% (14 of 14), after using LCLs from unaffected controls and patients with SMA and IBD (Table 1). The reprogramming efficiency for cell lines was between 0.001% and 0.1% (Table 1) and was consistent with reported efficiencies for episomal plasmid-based reprogramming.

**Table 1. Lymphoblastoid cell line-derived iPSC lines generated**

| **LCL-iPSC** | **Parent LCL** | **Associated disease/mutation** | **Reprogramming efficiency (%)** |
|---|---|---|---|
| 28iCTR-nxx | 120928 | Unaffected control | 0.001 |
| 36iCTR-nxx | 120036 | Unaffected control | 0.001 |
| 49iCTR-nxx | GM22649 | Unaffected control | 0.002 |
| 77iCTR-nxx | 121077 | Unaffected control | 0.001 |
| 87iCTR-nxx | GM23687A | Unaffected control | 0.06 |
| 688iCTR-nxx | GM23688 | Unaffected control | 0.004 |
| 14iIBD-nxx | 110414 | IBD | 0.002a |
| 20iIBD-nxx | 051720 | IBD | 0.002a |
| 44iIBD-nxx | 101414 | IBD | 0.002a |
| 71iIBD-nxx | 100771 | IBD | 0.002a |
| 87iIBD-nxx | 101787 | IBD | 0.002a |
| 428iIBD-nxx | 110428 | IBD | 0.002a |
| 55iSMA3-nxx | GM23255 | SMA | 0.004 |
| 84iSMA3-nxx | GM10684B | SMA | 0.006 |

| | | | |
|---|---|---|---|
| ^{a} This value is an underestimate because efficiency was calculated based on isolated clones only and not all detectable clones. Abbreviations: CTR, control; IBD, inflammatory bowel disease; iPSC, induced pluripotent stem cell; LCL, lymphoblastoid cell line; SMA, spinal muscular atrophy; xx, different clone numbers of iPSC lines. | | | |

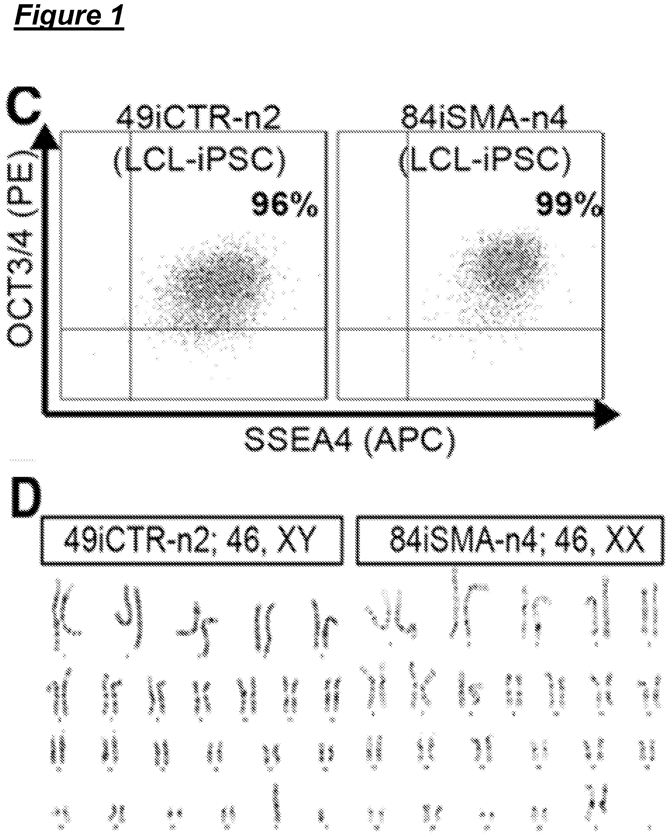

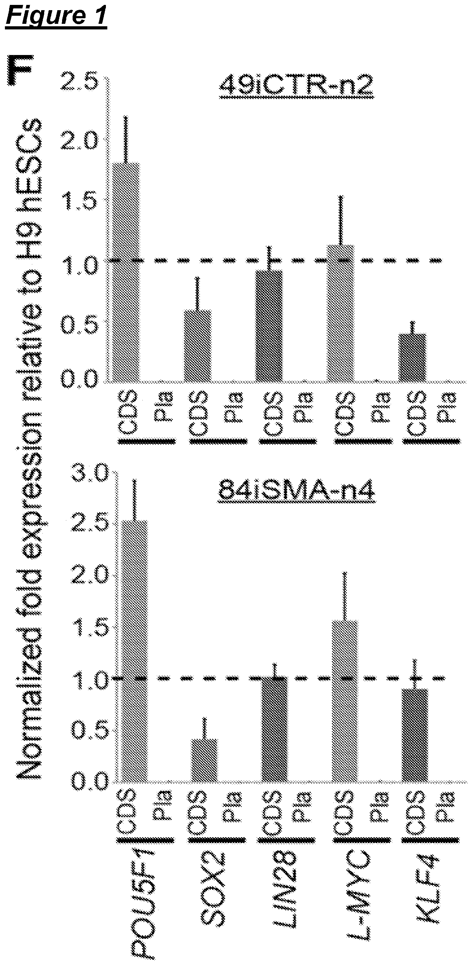

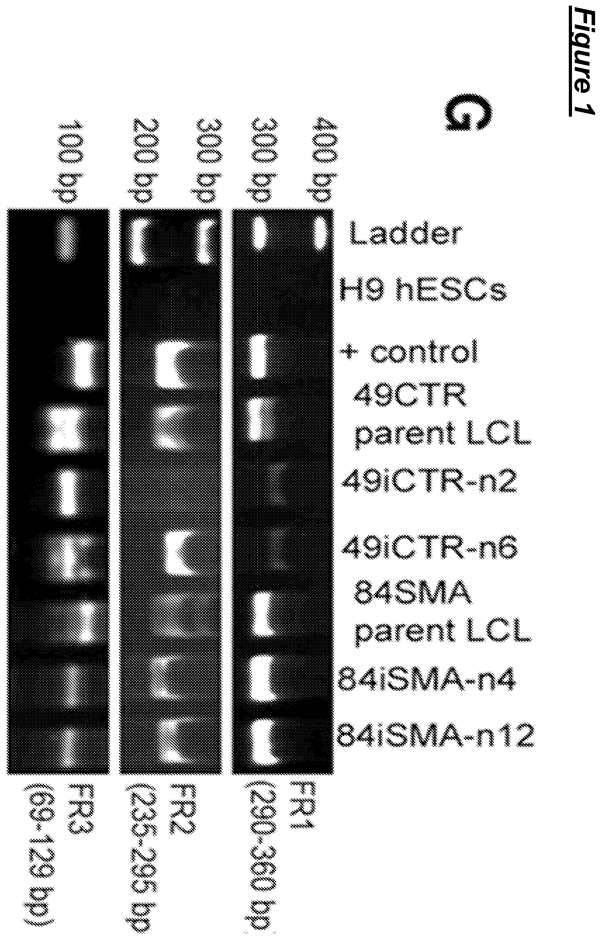

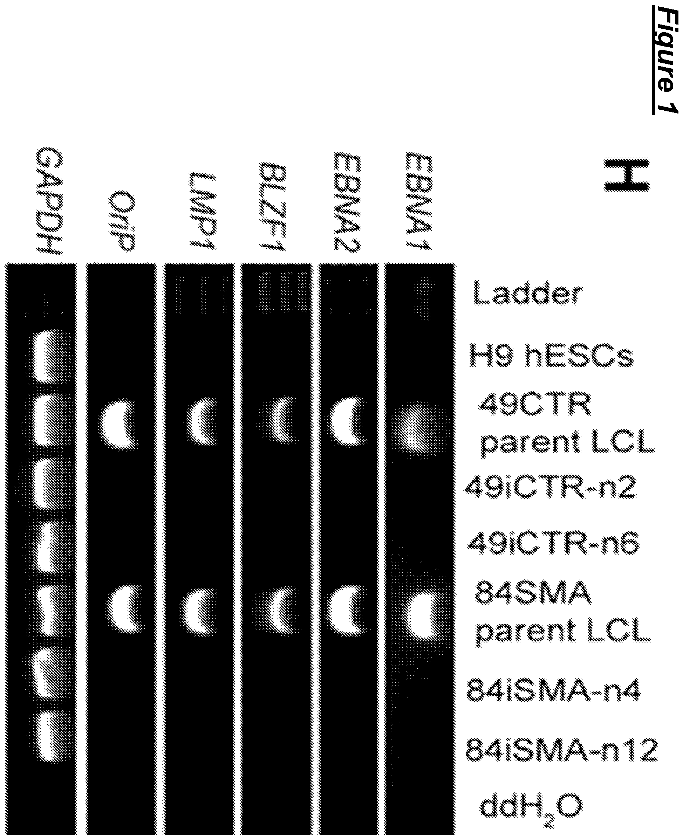

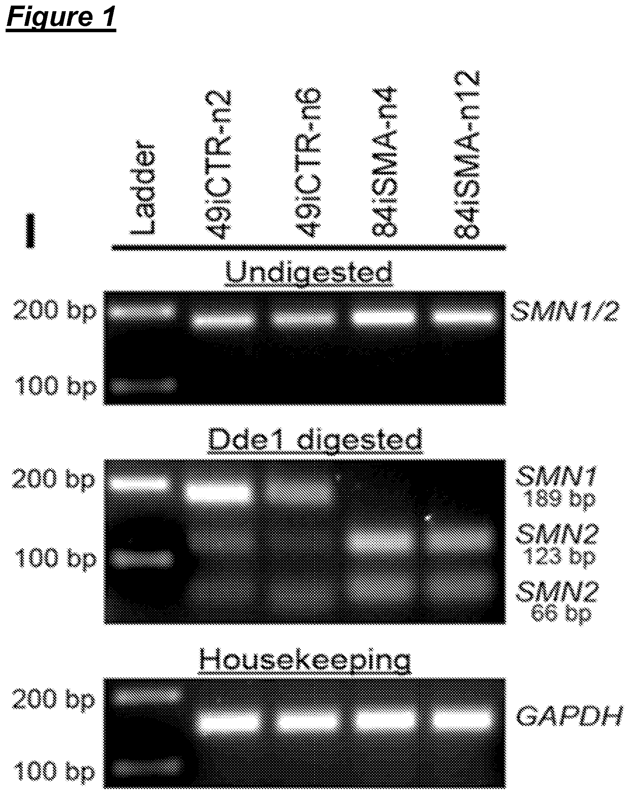

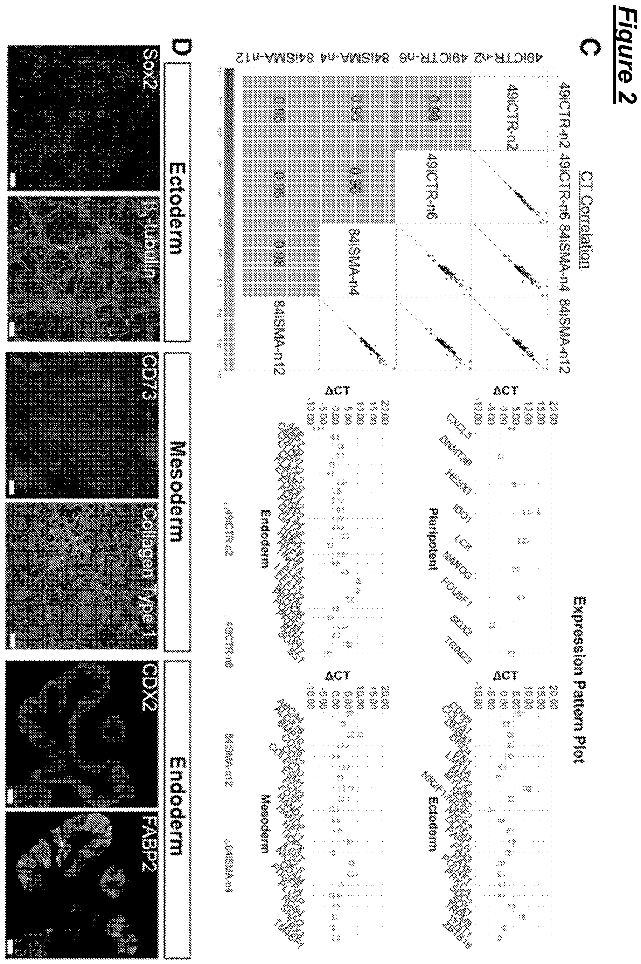

Representative results are highlighted here from independent clonal LCL-iPSC lines isolated using LCLs of a control individual (49iCTR-n2 and -n6) and a SMA patient (84iSMA-n4 and -n12). All LCL-iPSC lines exhibited typical PSC characteristics, including tightly packed colonies, high cell nuclear-cytoplasmic ratio, robust alkaline phosphatase activity, and production of surface and nuclear pluripotency proteins **(****Fig. 1B****).** Indeed, quantification revealed that .96% of cells expressed the pluripotency markers OCT4 and SSEA4 **(****Fig. 1C****).** They had a normal karyotype **(****Fig. 1D****)** and passed the PluriTest assay, demonstrating that the LCL-iPSC transcription profile was analogous to well established human embryonic stem cells and fib-iPSCs, but not differentiated fibroblasts and neural progenitor cells **(Fig. IE).** Expression of endogenous pluripotency genes and absence of exogenous transgenes was also demonstrated **(****Fig. 1F****)**, confirming the "footprint-free" status of LCL-iPSCs. Polymerase chain reaction (PCR) testing for immunoglobulin heavy chain rearrangements, which occur exclusively in committed B cells, confirmed that the LCL-iPSCs were clonal derivatives from their parental LCLs **(****Fig. 1G****).** Interestingly, all the EBV-related latency elements, necessary for transformation of resting Bcells to actively proliferating LCLs, were eventually eliminated from the established LCLiPSCs **(****Fig. 1H****).** Although it has been reported that iPSCs lose most of their gene expression and epigenetic profiles related to the original cell source, a critical aspect for disease modeling using iPSCs is that the disease genotype is maintained after reprogramming. PCR-restriction fragment-length polymorphism analysis of DNA from control and SMA patient LCL-iPSCs demonstrated that both the control and SMA lines maintained SMN2, whereas SMN1 was absent from the SMA lines **(****Fig. 1I****).** LCL-iPSCs spontaneously formed embryoid bodies (EBs) containing three germinal layers, as evidenced by downregulation of the pluripotency TDGF gene expression, and up-regulation of NCAM1 (ectoderm), HAND1 and MSX1 (mesoderm), and AFP (endoderm) genes, when compared with LCL-iPSCs **(****Fig. 2A****).** Furthermore, their equivalent trilineage potential was demonstrated using the new TaqMan human pluripotent stem cell Scorecard assay **(****Fig. 2B****).** It was observed that EB gene expression across all four LCL-iPSC lines had high similarity rates, illustrated in the pairwise correlation coefficient scatter and expression plots of the pluripotency and germ-layer gene groups **(****Fig. 2C****).** Subsequently, the ability of LCL-iPSCs to be patterned into particular cell types representative of each germ layer was determined by the addition of known morphogens, cytokines, and small molecules that promote specific germ-layer differentiation. All LCL-iPSCs could be induced to form neural ectoderm expressing Sox2 and b3-tubulin, cardiac mesoderm expressing CD73 and collagen type 1 that displayed a beating phenotype and intestinal organoid endoderm expressing CDX2-and FABP2-positive enterocytes **(****Fig. 2D****).**

Because LCL-iPSCs appeared to have similar characteristics to the fib-iPSCs, it was important to next determine whether they can similarly be directed to form disease-relevant cells. SMA is devastating childhood disease characterized by degeneration of lower spinal MNs, often resulting in death. Using a stepwise neuralization, caudalization, and ventralization process, the Inventors assessed whether LCL-iPSCs could be efficiently directed to produce iPSC-derived motoneurons ("i-MNs"). The Inventors first generated an expandable population of spinal MN precursor cells expressing the immature spinal MN transcription factors, Nkx6.1 and Islet1, which then reproducibly matured into i-MNs (50%-60%) expressing neurofilament, heavy chain (SMI32), and choline acetyltransferase **(****Fig. 3B****).** The early i-MNs derived from LCL-iPSCs also expressed Hb9, another well-described spinal MNspecific transcription factor **(****Fig. 4****).** The depletion of full-length SMN transcript (2.5- to 7-fold) and SMN protein in both clones of 84iSMA when compared with control 49iCTR cells confirmed that the SMA genotype was maintained in i-MNs, which is crucial for effective disease modeling **(****Fig. 3B****).**

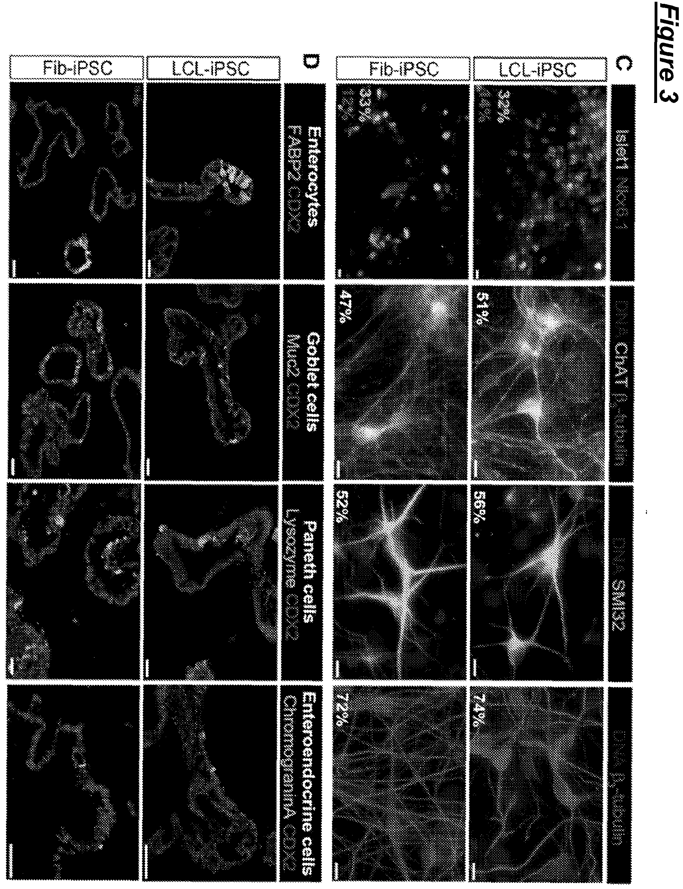

Finally, it is possible that the original donor cell type could prejudice the later differentiation potential of iPSCs because of residual epigenetic memory after reprogramming. Therefore, the Inventors assessed whether the morphology and efficiency of SMA and IBD disease-relevant cell types could be differentiated comparably from blood-derived LCL-iPSCs and fib-iPSCs. The Inventors observed appropriate human cellular subtypes of spinal MNs and intestinal organoids that were indistinguishable in morphology, growth rates, and cell numbers when directed from LCL-iPSCs or fibiPSCs **(****Fig.3C,3D****),** suggesting an analogous differentiation potential of all iPSCs with this reprogramming method, which is independent of the starting donor cell type.

### Example 23

### Conclusion

Given that numerous patient LCL repositories exist worldwide, it would greatly benefit disease modeling, drug screening, and regenerative medicine applications if these LCLs could be used to reliably generate iPSCs. As such, the Inventors report a method for reproducible generation of nonintegrating iPSCs from blood-derived LCLs using a novel episomal reprogramming strategy. Validation of these LCLiPSCs show that they are virtually indistinguishable from routinely used fibroblast-derived iPSCs. Importantly, the Inventors show that they can be differentiated into multiple disease-relevant cell types. Thus the use of abundantly available patient-specific LCLs linked with correlative genotype-phenotype data may be indispensable in determining underlying molecular mechanisms and discovering novel therapeutics for simple Mendelian or complex human diseases.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein.

## Claims

1. An in vitro method of generating lymphoblastoid cell line derived induced pluripotent stem cells, comprising:
providing a quantity of lymphoblastoid cell line cells (LCLs);
delivering a quantity of reprogramming factors into the LCLs, wherein the reprogramming factors comprise short hairpin RNAs targeting p53 ("shRNA-p53");
culturing the LCLs in a reprogramming media for at least 7 days, wherein the reprogramming media comprises small chemical induction molecules PD0325901, CHIR99021, HA-100, and A-83-01; and
further culturing the LCLs in an induction media for at least 10 days, wherein delivering the reprogramming factors, culturing and further culturing generates lymphoblastoid cell line derived induced pluripotent stem cells.

2. The method of claim 1, wherein delivering a quantity of reprogramming factors comprises nucleofection.

3. The method of claim 1, wherein the reprogramming factors further comprise one or more factors selected from the group consisting of: Oct-4, Sox-2, Klf-4, c-Myc, Lin-28, SV40 Large T Antigen ("SV40LT").

4. The method of claim 3, wherein the reprogramming factors are encoded in one or more oriP/EBNA1 derived vectors.

5. The method of claim 4, wherein the one or more oriP/EBNA1 derived vectors comprise pEP4 E02S ET2K, pCXLE-hOCT3/4-shp53-F, pCXLE-hSK, and pCXLE-hUL.

6. The method of claim 1, wherein culturing the LCLs in a reprogramming media is for:
(a) at least 7, 8, 9, 10, 11, 12, 13, 14, 15, or 16 days; or
(b) 8 to 14 days.

7. The method of claim 1, wherein further culturing the LCLs in an induction media is for at least 10, 11, 12, 13, 14, 15, 16, 17, 18, 19, 20, 21, 22, 23, 24, 25, 26, 27, 28, 29, or 30 days.

8. The method of claim 1, wherein the induction media is a serum-free media.

9. The method of claim 1, wherein the LCLs have been isolated from a subject possessing a disease mutation.

10. The method of claim 9, wherein the disease mutation is associated with a neurodegenerative disease, disorder and/or condition.

11. The method of claim 9, wherein the disease mutation is associated with an inflammatory bowel disease, disorder, and/or condition.

## Patentansprüche

1. In-vitro-Verfahren zum Erzeugen einer lymphoblastoiden Zellinie, die von induzierten pluripotenten Stammzellen abgeleitet ist, umfassend:
Bereitstellen einer Menge an Zellen lymphoblastoider Zellinien (LCLs); Abgeben einer Menge an Umprogrammierungsfaktoren in die LCLs, wobei die Umprogrammierungsfaktoren Short Hairpin RNAs targeting p53 ("shRNA-53") umfasst;
Kultivieren der LCLs in einem Umprogrammierungsmedium für zumindest 7 Tage, wobei das Umprogammierungsmedium kleine chemische Induktionsmoleküle PD0325901, CHIR99021, HA-100 und A-83-01 umfasst; und
weiteres Kultivieren der LCLs in einem Induktionsmedium für zumindest 10 Tage, wobei das Abgeben der Umprogrammierungsfaktoren, das Kultivieren und das weitere Kultivieren eine lymphoblastoide Zellinie erzeugt, die von induzierten pluripotenten Stammzellen abgeleitet ist.

2. Verfahren nach Anspruch 1, wobei das Abgeben einer Menge an Umprogrammierungsfaktoren eine Nukleofektion umfasst.

3. Verfahren nach Anspruch 1, wobei die Umprogrammierungsfaktoren weiter einen oder mehrere Faktoren umfassen, ausgewählt aus der Gruppe bestehend aus: Oct-4, Sox-2, Klf-4, c-Myc, Lin-28, SV40 Großes T-Antigen ("SV40LT").

4. Verfahren nach Anspruch 3, wobei die Umprogrammierungsfaktoren in einem oder mehreren oriP/EBNA1-abgeleiteten Vektoren codiert sind.

5. Verfahren nach Anspruch 4, wobei der eine oder die mehreren oriP/EBNA1-abgeleiteten Vektoren pEP4 E02S ET2K, pCXLE-hOCT3/4-shp53-F, pCXLE-hSK und pCXLE-hUL umfassen.

6. Verfahren nach Anspruch 1, wobei die Dauer des Kultivierens der LCLs in einem Umprogrammierungsmedium:
(a) zumindest 7, 8, 9, 10, 11, 12, 13, 14, 15 oder 16 Tage beträgt; oder
(b) 8 bis 14 Tage beträgt.

7. Verfahren nach Anspruch 1, wobei die Dauer des weiteren Kultivierens der LCLs in einem Induktionsmedium zumindest 10, 11, 12, 13, 14, 15, 16, 17, 18, 19, 20, 21, 22, 23, 24, 25, 26, 27, 28, 29 oder 30 Tage beträgt.

8. Verfahren nach Anspruch 1, wobei das Induktionsmedium eine serumfreies Medium ist.

9. Verfahren nach Anspruch 1, wobei die LCLs aus einem Subjekt isoliert wurden, das eine Krankheitsmutation aufweist.

10. Verfahren nach Anspruch 9, wobei die Krankheitsmutation mit einer neurodegenerativen Erkrankung, Störung und/oder Zustand verbunden ist.

11. Verfahren nach Anspruch 9, wobei die Krankheitsmutation mit einer entzündlichen Darmerkrankung, Störung und/oder Zustand verbunden ist.

## Revendications

1. Procédé in vitro de génération d'une lignée cellulaire de lymphoblastoïdes dérivée de cellules souches pluripotentes induites, comprenant :
l'apport d'une certaine quantité de cellules de lignée cellulaire de lymphoblastoïdes (LCL) ; la livraison d'une certaine quantité de facteurs de reprogrammation au sein des LCL, dans lequel les facteurs de reprogrammation comprennent des courts ARN en épingle à cheveux ciblant le p53 (« shRNA- p53 ») ;
la mise en culture des LCL dans un milieu de reprogrammation pendant au moins 7 jours, dans lequel le milieu de reprogrammation comprend les petites molécules d'induction chimique PD0325901, CHIR99021, HA-100, et A-83-01 ; et
la mise en culture supplémentaire des LCL dans un milieu d'induction pendant au moins 10 jours, dans lequel la livraison des facteurs de reprogrammation, la mise en culture et la mise en culture supplémentaire génèrent la lignée cellulaire de lymphoblastoïdes dérivée de cellules souches pluripotentes induites.

2. Procédé selon la revendication 1, dans lequel la livraison d'une certaine quantité de facteurs de reprogrammation comprend la nucléofection.

3. Procédé selon la revendication 1, dans lequel les facteurs de reprogrammation comprennent en outre au moins un facteur choisi dans le groupe constitué de : Oct-4, Sox-2, Klf-4, c-Myc, Lin-28, l'antigène T large du SV40 (« SV40LT »).

4. Procédé selon la revendication 3, dans lequel les facteurs de reprogrammation sont codés dans au moins un vecteur dérivé de oriP/EBNA1.

5. Procédé selon la revendication 4, dans lequel l'au moins un vecteur dérivé de oriP/EBNA1 comprend pEP4 E02S ET2K, pCXLE-hOCT3/4-shp53-F, pCXLE-hSK, et pCXLE-hUL.

6. Procédé selon la revendication 1, dans lequel la mise en culture des LCL dans un milieu de reprogrammation se fait pendant :
(a) au moins 7, 8, 9, 10, 11, 12, 13, 14, 15, ou 16 jours ; ou
(b) de 8 à 14 jours.

7. Procédé selon la revendication 1, dans lequel la mise en culture supplémentaire de LCL dans un milieu d'induction se fait au moins pendant 10, 11, 12, 13, 14, 15, 16, 17, 18, 19, 20, 21, 22, 23, 24, 25, 26, 27, 28, 29, ou 30 jours.

8. Procédé selon la revendication 1, dans lequel le milieu d'induction est un milieu exempt de sérum.

9. Procédé selon la revendication 1, dans lequel les LCL ont été isolées à partir d'un sujet possédant une mutation de maladie.

10. Procédé selon la revendication 9, dans lequel la mutation de maladie est associée à une maladie, un trouble et/ou une pathologie neurodégénérative.

11. Procédé selon la revendication 9, dans lequel la mutation de maladie est associée à une maladie, un trouble et/ou une pathologie inflammatoire de l'intestin.
